# EUROPÄISCHE PATENTSCHRIFT

(11) **EP 2 830 504 B1**
(45) Veröffentlichungstag und Bekanntmachung des Hinweises auf die Patenterteilung: **08.05.2019**
(21) Anmeldenummer: 13716197.2
(22) Anmeldetag: 22.03.2013
(51) Int. Cl.: A61B 6/03, A61B 6/00

(54) **PHASENKONTRAST-RÖNTGEN-TOMOGRAPHIEGERÄT**
PHASE CONTRAST X-RAY TOMOGRAPHY DEVICE
APPAREIL DE TOMOGRAPHIE À RAYONS X À CONTRASTE DE PHASE

(30) Priorität: 25.03.2012 DE 102012005767
(43) Veröffentlichungstag der Anmeldung: 04.02.2015
(73) Patentinhaber: ARP Angewandte Radiologische Physik UG, 71735 Eberdingen-Nussdorf (DE)
(72) Erfinder: BAUER, Walter, 71735 Eberdingen-Nussdorf (DE)
(74) Vertreter: Ostertag & Partner Patentanwälte mbB
(86) Internationale Anmeldenummer: PCT/EP2013/000873
(87) Internationale Veröffentlichungsnummer: WO 2013/143672

(56) Entgegenhaltungen:
- WO-A1-2009/115966
- US-A- 4 352 021
- US-A1- 2003 235 271
- US-A1- 2009 154 640

## Beschreibung

Die Erfindung betrifft ein Phasenkontrast-Röntgen-Tomographiegerät.

Gegenstände kann man in Durchstrahlung sowohl dadurch entdecken, dass die einfallende Strahlung im Gegenstand abgeschwächt wird, oder auch dadurch, dass die einfallende Strahlung im Gegenstand eine andere Fortpflanzungsgeschwindigkeit hat als außerhalb desselben. Für die Erkennung von Strukturen innerhalb eines Gegenstandes gilt das oben Gesagte gleichermaßen.

Änderungen in der Fortpflanzungsgeschwindigkeit führen zu Änderungen in der Phasenlage der Wellenfronten hinter dem Gegenstand. Typische Verfahren zum Erkennen der Änderung von Phasenlagen liegen darin, dass man mit dem Licht, welches den Gegenstand durchquert hat, Interferenzfiguren erzeugt und deren Helligkeitsverteilung mit geeigneten Detektoren ausmisst.

Derartige Detektoren sind heute insbesondere Zeilendetektoren, welche eine Vielzahl in engem Abstand regelmäßig in Detektorlängsrichtung aufeinanderfolgende Detektionspixel aufweisen. Die Ausgangssignale derartiger Detektoren können unter Anwendung bekannter Techniken geformt und ausgewertet werden.

Verwendet man anstelle von Zeilendetektoren Streifendetektoren, die auch in einer zur Detektorlängsrichtung senkrechten Richtung regelmäßig unter kleinem Abstand aufeinanderfolgende Detektorelemente aufweisen, deren Anzahl allerdings kleiner ist als in Detektorlängsrichtung, so kann man die Interferenzfiguren auch in einer zweiten Richtung auswerten, wodurch sich die Qualität des Bildes verbessern lässt oder die zur Aufnahme des Bildes notwendige Zeit verringern lässt. Vereinfachend sollen hier unter Zeilendetektoren auch Streifendetektoren verstanden werden, um Doppelnennungen zu vermeiden, wo nicht anders gesagt.

Die soeben angesprochene Möglichkeit der Erkennung von Strukturen eines Gegenstandes unter Ausnützung der Tatsache, dass Licht in unterschiedlichen Materienbereichen unterschiedliche Lichtgeschwindigkeit aufweist, wird auch Phasenkontrastverfahren genannt, da man eine kontrastreiche Darstellung des Gegenstandes aus Phasenunterschieden in der Lage der Wellenfronten herleitet.

Phasenkontrastverfahren sind insbesondere da von großem Interesse, wo Materialien Licht nur wenig absorbieren, also weitgehend für das verwendete Licht durchlässig sind.

Solche Verhältnisse liegen insbesondere im Bereich der Röntgen-Durchstrahlungsbilder vor, da Röntgenstrahlen, wie sie üblicherweise in der medizinischen Diagnostik und der Materialprüfung verwendet werden, nur wenig von Materialien absorbiert werden, wie sie in organischen Geweben vorkommen. Bekanntlich hängt die Absorption von Röntgenstrahlen in Materie sehr stark von der Kernladungszahl der dort angefundenen Atome ab. Organische Materie ist aber überwiegend aus Wasserstoffatomen, Kohlenstoffatomen, Stickstoffatomen und Sauerstoffatomen aufgebaut, alles Elemente, die eine kleine Kernladungszahl aufweisen.

In der DE 10 2006 046 034 A1 ist schon ein Phasenkontrast-Röntgen-Tomographiegerät beschrieben, welches nach dem eingangs geschilderten Prinzip arbeitet. Bei diesem bekannten Röntgen-Tomographiegerät erfolgt die Erzeugung des Interferenzmusters durch zwei in unterschiedlichem Abstand fluchtend vor einem linearen Detektor angeordnete Röntgengitter. Im Einzelnen geht es in der DE 10 2006 046 034 A1 darum, ein Phasenkontrast-Röntgen-Tomographiegerät anzugeben, welches gleichzeitig auch für klassische Röntgenaufnahmen verwendet werden kann, die auf der unterschiedlichen Absorption der Röntgenstrahlen in unterschiedlichen Bereichen des Untersuchungsgegenstandes beruhen.

Bei dem bekannten Röntgen-Tomographiegerät sind auf einer Kreisführung (Gantry) einander diametral gegenüberliegend eine Röntgenquelle und eine Phasenmesseinrichtung angeordnet. Sie laufen in starrer Relativlage um die Achse der Gantry um. Diese Achse wird nachstehend auch einfach als Geräteachse bezeichnet.

Beim Umlaufen von Röntgenquelle und Phasenmesseinrichtung wird ein auf der Geräteachse angeordneter zu untersuchender Körper, z.B. ein Patient, aus unterschiedlichen Richtungen durchstrahlt. Der zur Durchstrahlung verwendete Röntgenstrahl hat in Richtung der Geräteachse nur geringe Abmessungen, hat also die Gestalt eines sehr flachen Fächers. Bei einem Umlauf von Röntgenquelle und Phasenmesseinrichtung wird somit eine Schicht des zu untersuchenden Objekts erfasst. Dadurch, dass man eine Relativbewegung zwischen der Gantry und dem zu untersuchenden Objekt in axialer Richtung erzeugt, kann man eine Vielzahl eng benachbarter Schnitte durch das Objekt legen.

Aufgrund der mechanischen Trägheit der umlaufenden Geräteteile ist die zeitliche Auflösung des bekannten Tomographen gering. Sich bewegende Strukturen wie z.B. der pulsierende Herzmuskel können so nicht aufgelöst werden.

Bei der Phasenkontrast-Röntgen-Computertomographie müssen in der Phasenmesseinrichtung Röntgengitter mit sehr kleinen Gitterkonstanten verwendet werden. Diese Röntgengitter müssen extrem genau positioniert werden und darüber hinaus auch noch sehr präzise um kleine Strecken verschoben werden, um gezielte kleine Änderungen in den Interferenzmustern zu erzeugen, die für die Auswertung der Interferenzmuster wichtig sind. Damit muss das bekannte Tomographiegerät sehr stabilen Aufbau aufweisen, um erschütterungsbedingte oder verzugsbedingte Änderungen im Interferenzmuster sicher auszuschließen. Da die Gantry einen Untersuchungsbereich umgeben muss, der typischerweise deutlich größer ist als die Abmessungen eines menschlichen Körpers, sind solche Tomographiegeräte auch sehr sperrig.

Ein ähnliches Phasenkontrast-Röntgen-Tomographiegerät mit umlaufender Gantry ist aus der WO 2009/115966 A1 bekannt.

Es sind auch Röntgen-Tomographiegeräte bekannt, bei denen ein umlaufender Röntgenfächer dadurch erzeugt wird, dass man einen durch eine Ablenkspule gedrehten (= auf einer Kreisbahn bewegten) Elektronenstrahl, der von einer Elektronenkanone des Gerätes bereitgestellt wird, auf ein ringförmiges Target fokussiert, welches in der Gantry feststehend angeordnet ist. Ein derartiges Tomographiegerät ist in der US 4 521 901 beschrieben. Hier hat man also keine schweren und trägen umlaufenden Geräteteile, und mit einem solchen Gerät können auch in Bewegung befindliche Organe untersucht werden.

Dieses Gerät arbeitet aber in Absorption, erzeugt also klassische Röntgen-Tomogramme.

Ein ähnliches Röntgen-Tomographiegerät für klassische Röntgen-Tomographie ist aus der US 4, 352, 021 A bekannt.

Durch die vorliegende Erfindung soll ein verbessertes Phasenkontrast-Röntgen-Tomographiegerät geschaffen werden.

Diese Aufgabe ist erfindungsgemäß gelöst durch ein Phasenkontrast-Röntgen-Tomograpiegerät, welches die im Anspruch 1 aufgeführten Merkmale aufweist.

Das erfindungsgemäße Phasenkontrast-Röntgen-Tomographiegerät lässt sich weitestgehend ohne bewegte mechanische Teile aufbauen. Denn die Röntgenlichtquelle mit dem Elektronenstrahl, der auf einer Bahn über ein Target bewegt wird, erlaubt es, ohne mechanisch gedrehte Komponenten einen umlaufenden Röntgenstrahl zu erzeugen. Insbesondere sind Quelle, Gitter und Detektor mechanisch feststehend, wodurch gewährleistet ist, dass die Phasenmesseinrichtung hohe Stabilität und hohe Konstanz ihres Arbeitsverhaltens gewährleistet.

Vorteilhafte Weiterbildungen der Erfindung sind in den Unteransprüchen angegeben.

Die Weiterbildung der Erfindung gemäß Anspruch 2 ist im Hinblick auf eine Effektmodulierung und eine gute Signalaufbereitung in den an die Detektorzeile angeschlossenen Signalkanälen von Vorteil.

Bei einem Tomographiegerät, bei welchem der Elektronenstrahl wegabhängig ein- und ausgeschaltet wird, und/oder bei welchem der Elektronenstrahl intermittierend, vorzugsweise um gleiche Inkremente bewegt wird, verweilt der Röntgenfächer immer eine kurze Zeitspanne in gleicher Relativposition zum Objekt und wird dann jeweils um ein Inkrement weitergeschaltet. Dies ist im Hinblick auf rauscharme und kontrastreiche Bilder von Vorteil.

Bei einem Tomographiegerät gemäß Anspruch 3 lässt sich der Fokuspunkt des Elektronenstrahles auf dem Target und damit der Ursprung des Röntgenstrahles so variieren, dass die Breite des Röntgenstrahl-Fächers eingestellt werden kann.

Bei einem Tomographiegerät gemäß Anspruch 4 kann der Fokus des Elektronenstrahles und damit der Ausgangspunkt des Röntgenstrahles auf einem Kreis bewegt werden. Damit ist es möglich, ein Objekt aus unterschiedlichen Richtungen im Wesentlichen senkrecht zu einer Objektachse mit Röntgenlicht zu beaufschlagen. Ist dabei das Target kegelstumpfförmig, geht mit der radialen Verlagerung des Fokuspunktes des Elektronenstrahles zugleich eine axiale Verlagerung desselben einher. Auf diese Weise kann man auch die Lage des Röntgenstrahles in axialer Richtung variieren. Hierdurch kann auf eine gegebenenfalls zur Phasenmessung notwendige axiale Bewegung der Röntgenlicht-Beugungsstrukturen verzichtet werden.

Dabei ist die bevorzugte Weiterbildung, bei welcher der Öffnungswinkel des Target-Kegels so gewählt ist, dass er zu einem Talbot-Interferometer der Phasenmesseinrichtung passt, im Hinblick auf niedrige Strahlendosis und auf die notwendige räumliche Kohärenz für die Ausbildung kontrastreicher Interferenzstreifen von Vorteil.

Bei einem Tomographiegerät, bei welchem ein radial außen liegender oder ein radial innen liegender Rand des Targets eine im Wesentlichen axial in Richtung auf eine Elektronenstrahlquelle der Röntgenlichtquelle zurücklaufende Filterwand trägt, die vorzugsweise von dem Target oder einer letzteres tragenden Gehäusewand getragen ist, wobei letztere vorzugsweise Röntgenstrahlung stark absorbiert, vorzugsweise sperrt, wird ein Teil des Röntgenspektrums durch eine Filterwand zurückgehalten, bevor der Röntgenstrahl auf das zu untersuchende Objekt trifft. Dies ist im Hinblick auf Röntgenstrahlerzeugung mit hoher Leistungsdichte und auf kontrastreiche Interferenzmuster von Vorteil.

Eine Weiterbildung der Erfindung, bei welcher das Target der Röntgenlichtquelle einen Grundkörper aufweist, der aus einem Grundmaterial mit niederer Kernladungszahl und vorzugsweise hoher Wärmeleitfähigkeit hergestellt ist, und dass das Target eine einer Elektronenstrahlquelle der Röntgenlichtquelle zugewandte Funktionsschicht aufweist, die aus einem Material mit hoher Kernladungszahl gefertigt ist, welches vorzugsweise gute Wärmeleitfähigkeit aufweist und/oder einem Talbot-Interferometer angepasst ist, ist im Hinblick auf eine effektive Röntgenstrahlungserzeugung bei gleichzeitig geringem Gewicht des Gerätes von Vorteil. Die Funktionsschicht kann dabei eine sehr dünne Schicht aus Funktionsmaterial sein, deren Dicke vorzugsweise der Anodenspannung und der Eindringtiefe des Elektronenstrahles angepasst ist und nochmals vorzugsweise weniger als 20 µm, vorzugsweise weniger 10 µm beträgt und die vorzugsweise durch Aufdampfen oder Sputtern hergestellt ist. Dies ist im Hinblick auf gute Röntgenstrahlerzeugung einerseits und gute Wärmeabfuhr andererseits von Vorteil.

Üblicherweise arbeitet die hier verwendete Phasenmesseinrichtung nur in einem engen Wellenlängenbereich des Röntgenlichts. Daher ist eine Weiterbildung der Erfindung, bei welcher das Target eine einer Elektronenstrahlquelle der Röntgenlichtquelle zugewandte Funktionsschicht aufweist, die aus einem Material gefertigt ist, das die Erzeugung von Röntgenlicht erlaubt, dessen Spektrum bei einer Wellenlänge einen Peak aufweist, die an ein Talbot-Interferometer der Phasenmesseinrichtung angepasst ist, vorteilhaft. Denn dann sind die Phasenmesseinrichtung und das für das Target verwendete Funktionsmaterial derart aufeinander abgestimmt, dass zumindest eine der materialspezifischen, scharfen Linien des Röntgenspektrums der Röntgenlichtquelle bei einer Wellenlänge liegt, für welche die Phasenmesseinrichtung optimiert ist. Auch die Röntgeneigenschaften des zu untersuchenden Objekts sind bei der Auswahl des Funktionsmaterials maßgeblich.

Ein Tomographiegerät mit den im Anspruch 5 angegebenen Merkmalen eignet sich besonders gut für eine Untersuchung eines Objektes von allen Seiten her. Dabei ist die Kreisform oder Polygonform im Hinblick auf die Erzeugung vollständiger Daten und die Sicherstellung weitgehend identischer Untersuchungsbedingungen in Umfangsrichtung von Vorteil, aber nicht zwingend notwendig.

Bei einem Tomographiegerät gemäß Anspruch 6 kann man ein in der Phasenmesseinrichtung erzeugtes Interferenzbild in zwei zueinander senkrechten Richtungen auswerten. Dies ermöglicht die Phasenmessung in einem aufgelösten Bildpixel mit nur einer Aufnahme ohne die Einbeziehung von zu vielen Detektorpixeln in Umfangsrichtung. Die Aufnahmegeschwindigkeit kann so gesteigert werden, ohne die Auflösung in Umfangsrichtung zu verschlechtern.

Die Weiterbildung der Erfindung gemäß Anspruch 7 macht es möglich, im Tomographiegerät auch eine Röntgenlichtquelle einzusetzen, die gewöhnliche Fokusgrößen besitzt, also ursprünglich nicht ausreichend räumlich kohärentes Röntgenlicht erzeugt. Durch das hinter der Röntgenlichtquelle angeordnete Röntgengitter wird von dem von der Röntgenlichtquelle erzeugten Röntgenlicht ein näherungsweise kohärenter Anteil abgespalten, der dann für die Phasenkontrast-Untersuchung verwendet wird.

Die Weiterbildung der Erfindung gemäß Anspruch 8 ermöglicht die doppelte Nutzung des Röntgengitters sowohl als Kohärenzgitter als auch als Phasenmessgitter. Denn bei einem zumindest abschnittsweise, vorzugsweise vollständig, den Untersuchungsbereich umgebenden Röntgengitter kann das vom Brennfleck emittierte Röntgenlicht das Röntgengitter zunächst im Wesentlichen radial von außen nach innen durchlaufen, um für die Phasenkontrastmessung ausreichend kohärentes Röntgenlicht zu erzeugen. Nach dem Durchlaufen des Untersuchungsbereiches tritt das Röntgenlicht auf der diametral gegenüberliegenden Seite des Untersuchungsbereichs in umgekehrter Richtung, d.h. radial von innen nach außen, durch das Röntgengitter hindurch, sodass dieses als Röntgenlicht-Beugungsstruktur zur Vermessung der Phasenlage dient. Dies erlaubt die Verwendung nur eines Röntgengitters falls die Detektorauflösung ausreichend ist, um die bei der Vermessung der Phasenlage entstehenden Interferenzmuster zu erfassen. Andernfalls muss noch eine weitere Röntgenlicht-Beugungsstruktur vorgesehen werden.

Die Weiterbildungen der Erfindung gemäß den Ansprüchen 9 und 10 sind im Hinblick auf die Ausbildung eines kontrastreichen Interferenzmusters in der Phasenmesseinrichtung von Vorteil.

Eine Weiterbildung der Erfindung, bei welcher die Interferenzeinrichtung auf den Schwerpunkt oder einen gewünschten Spektralbereich des Spektrums der Röntgenlichtquelle angepasst ist, ist dabei im Hinblick auf gute Empfindlichkeit und Kontrastauflösung des Tomographiegerätes von Vorteil.

Bei einem Tomographiegerät, bei welchem die Interferenzeinrichtung zugleich als Filter für langwellige Bereiche des Spektrums der Röntgenlichtquelle ausgebildet ist, werden langwellige Bereiche des Spektrums der Röntgenlichtquelle abgespalten, welche das Phasenkontrastbild nachteilig als Untergrund beeinflussen könnten.

Auch die Weiterbildung der Ansprüche 11 ist im Hinblick auf die Untersuchung eines Objektes in unterschiedlichen radialen Richtungen von Vorteil.

Gleiches gilt für eine Weiterbildung, bei welcher mindestens eine der Röntgenlicht-Beugungsstrukturen in Richtung einer Geräteachse periodisch ist, und/oder bei welcher mindestens eine der Röntgenlicht-Beugungsstrukturen in Umfangsrichtung periodisch ist.

Die Weiterbildung der Erfindung gemäß Anspruch 12 gestattet es, das Target, die erste Interferenzeinrichtung und gegebenenfalls auch noch eine Filterwand in einem einzigen Bauteil zu vereinen. Zusätzlich ist hierdurch auch eine genau in der Targetebene liegende Durchstrahlung ohne axialen Versatz möglich, wodurch eine höhere Bildqualität erzielt werden kann.

Eine Weiterbildung der Erfindung, bei welcher das ein Objekt durchstrahlende Röntgenlicht im Wesentlichen radial verläuft und durch die durchstrahlten Bereiche der Filterwand und durch die erneut durchdrungenen Materialien des auf der gegenüberliegenden Seite liegenden Abschnitts des Target eine weitere Filterung des Energiespektrums erfährt, gestattet ebenfalls eine genau in der Targetebene liegende Durchstrahlung, wobei alle auf dem Weg zum Detektor durchstrahlten Materialien in ihrer Absorptions- und Filterrichtung in Objektrichtung und gegen die Objektrichtung optimiert werden können.

Die Weiterbildung der Erfindung gemäß Anspruch 13 gestattet es, die Interferenzmuster bei vorbekannten kleinen Änderungen in den Relativpositionen von Lichtquelle, Beugungsstrukturen und Detektorzeile zu variieren und aus den kleinen Änderungen der Interferenzstreifen bei bekannten Relativbewegungen die Phasenlage des Röntgenlichtes in der Phasenmesseinrichtung zu bestimmen. Hierdurch wird es gegenüber dem Stand der Technik ermöglicht, die Phasenlage des Röntgenlichts zeitlich nacheinander und dafür mit einer der Größe der Detektorpixel entsprechenden genauen Ortsauflösung zu bestimmen.

Die Mittel zum Verändern einer oder mehrerer Relativlagen können einen Linearantrieb, einen Drehantrieb oder einen Kippantrieb umfassen.

Die Mittel zum Verändern einer oder mehrerer Relativlagen können einen Mikroaktor, zum Beispiel einen Piezoaktor, einen magnetostriktiven Aktor oder einen Elektret-Aktor, umfassen.

Die Mittel zur Veränderung einer oder mehrerer Relativlagen können Mittel zum Ändern der Beugungs-Geometrie einer Röntgenlicht-Beugungsstruktur umfassen.

Die vorgenannten Mittel stellen konkrete Mittel dar, wie man die Änderung der Interferenzbedingungen präzise und reproduzierbar ändert und so die Phasenmessung nun zeitlich nacheinander räumlich exakt auf eine Pixelgröße aufgelöst bestimmt werden kann, anstatt mit mehreren Detektorpixeln gleichzeitig die Phasenmessung vorzunehmen.

Eine Weiterbildung, bei welcher ein einer Bahnebene des Röntgenlichts benachbarter Bereich des Vakuumgefäßes für Röntgenlicht zumindest teilweise durchlässig ist und bei welcher die Röntgen-Phasenmesseinrichtung zumindest teilweise, vorzugsweise ganz radial außerhalb des Vakuumgefäßes (24) angeordnet ist, ist im Hinblick darauf vorteilhaft, den Röntgenstrahl bezogen auf die Geräteachse exakt horizontal zu führen. Bei einem solchen Röntgentomographiegerät sind zumindest Teile der Phasenmesseinrichtung auch gut zugänglich, was ihre Justierung erleichtert.

Durch die Weiterbildung der Erfindung gemäß Anspruch 14 erhält der Röntgenstrahl in axialer Richtung des Gerätes kleine Abmessung. Man kann so entsprechend dünne Schichten des untersuchten Objektes (Werkstück, Patient) untersuchen. Hierdurch kann auch die Dosisbelastung eines Patienten reduziert werden. Außerdem kann auf diese Weise die benötigte Gitter- und Detektorausdehnung in axialer Richtung deutlich gesenkt werden, wodurch die Kosten für die Herstellung reduziert werden.

Bei einem Tomographiegerät gemäß Anspruch 15 ist der Röntgenfächer auch in Umfangsrichtung begrenzt.

Eine solche Begrenzung erhält man, indem die Blendenfenster durch in Umfangsrichtung äquidistant aufeinanderfolgende Blendenkörper gebildet sind, die vorzugsweise stabförmig ausgebildet sind, auf mechanisch besonders einfache Weise, da die Blenden nicht umlaufend zu sein brauchen. In Zusammenhang mit dieser Weiterbildung wird dann der Elektronenstrahl und damit der durch diesen erzeugte Röntgenstrahl inkrementweise weiterbewegt, derart, dass in jedem Zyklus das nächste der durch die in Umfangsrichtung aufeinanderfolgenden Blenden begrenzte Fenster erreicht wird.

Bei einem Tomographiegerät, bei welchem die Inkremente dem Abstand der Blendenfenster angepasst sind, macht man von der inkrementweisen Weiterbewegung des Elektronenstrahles dazu Gebrauch, den durch den Elektronenstrahl erzeugten Röntgenfächer nacheinander in die verschiedenen Fenster des Blendenkörpers zu stellen.

Mit einer Weiterbildung der Erfindung, bei welcher die Teilung der Bahn des Elektronenstrahles gegenüber der Teilung der Blendenfenster um eine Phasenversetzung phasenversetzt ist, ist es möglich, die Achse des Röntgenfächers aus einer exakt radialen Richtung heraus zu schwenken. Dies ermöglicht, indem die Phasenversetzung wegabhängig so gewählt ist, dass ein vorgegebener Teilbereich eines Objektes durchstrahlt wird, einen Teilbereich eines Objektes zu durchstrahlen.

Ferner kann man eine in Umfangsrichtung wirkende weitere Blende, welche synchron zum Röntgenlicht um die Geräteachse umläuft und vorzugsweise Röntgenlicht mit in Umfangsrichtung kleinem Umfangs-Öffnungswinkel vorgibt, verwenden, um den Röntgenstrahl in der Fächerebene seitlich zu begrenzen. Die Verwendung umlaufender Blenden hat dabei den Vorteil, dass man die genannte Abmessung des Röntgenstrahles je nach Anwendungsfall in einem größeren Bereich variieren kann.

Eine Weiterbildung der Erfindung, bei welcher der Austrittsspalt und die Lage des Brennfleckes des Elektronenstrahles der Röntgenlichtquelle axial gegeneinander versetzt sind, wobei der Austrittsspalt vorzugsweise mit einer Filterwand kombiniert ist und nochmals vorzugsweise als Vakuumfenster ausgebildet ist, gestattet es, eine Detektorzeile oder einen Dektorstreifen außerhalb eines Gehäuses des Tomographiegerätes anzuordnen, da durch die geneigte Strahlführung der an einem Umfangspunkt des Gehäuses abgegebene Röntgenfächer auf seinem Weg zum Objekt und durch dieses hindurch eine so große axiale Lageänderung erfährt, dass er an dem dem Strahlquellpunkt gegenüberliegenden Bereich des Gerätegehäuses vorbeiläuft.

Nachstehend wird die Erfindung anhand von Ausführungsbeispielen unter Bezugnahme auf die Zeichnung näher erläutert. In dieser zeigen:
- Figur 1: eine schematische Darstellung, anhand derer die Wirkungsweise der Phasenkontrast-Röntgentomographie anhand eines einfachen Beispieles erläutert wird, wobei ein Interferenzmuster dargestellt wird, wie es von einer quadratischen Prüfstruktur mit trapezförmigem Querschnitt erzeugt wird;
- Figur 2: einen schematischen axialen Schnitt durch ein Phasenkontrast-Röntgen-Tomographiegerät;
- Figur 3: einen axialen Halbschnitt durch ein abgewandeltes Phasenkontrast-Röntgen-Tomographiegerät, welches mehrere Elektronenkanonen besitzt und für lange Objekte geeignet ist.
- Figur 4: eine axiale Aufsicht auf Strahlbegrenzungen und Röntgengitter eines Phasenkontrast-Röntgen-Tomographiegerätes, wobei einige Elemente in die Zeichenebene umgeklappt sind (oberer Abschnitt der Figur) sowie eine seitliche Ansicht des Tomographiegerätes (unterer Figurenteil);
- Figur 5: einen axialen Teilschnitt durch Targetring und Beugungsgitter eines weiteren Phasenkontrast-Röntgen-Tomograpiegerätes;
- Figur 6: eine vergrößerte Ansicht des Targetringes nach Figur 5;
- Figuren 7 bis 11: verschiedene Interferenzmuster, die sich für unterschiedliche Gitterkonstanten der Röntgengitter und unterschiedliche Verkippung derselben gegeneinander ergeben;
- Figur 12: eine perspektivische Ansicht einer ersten rotationssymmetrischen Röntgengitterstruktur;
- Figur 13: eine perspektivische Ansicht eines Segmentes einer Polygon-Röntgengitterstruktur;
- Figur 14: eine perspektivische Ansicht einer weiteren Polygon-Röntgengitterstruktur;
- Figur 15: ein Segment einer weiteren rotationssymmetrischen Röntgengitterstruktur;
- Figur 16: eine perspektivische Ansicht einer weiteren rotationssymmetrischen Röntgengitterstruktur, welche aus bogenförmigen Segmenten zusammengesetzt ist;
- Figur 17: eine ähnliche Ansicht wie Figur 15, wobei je doch die Durchlassbereiche der Gitterstruktur längs leicht geneigter Wendellinien angeordnet sind;
- Figur 18: eine perspektivische Ansicht einer makroskopisch zylindrischen Röntgengitterstruktur mit wendelförmig verlaufenden Strichen;
- Figur 19: eine ähnliche Ansicht wie Figur 18, wobei jedoch die Segmente der Röntgengitterstruktur geradlinig sind;
- Figur 20: eine perspektivische Ansicht einer PolygonRöntgengitterstruktur, wie sie in Figur 19 dargestellt ist, wobei diese aber zusätzlich noch von einer polygonförmigen teilweise mehrzeiligen Detektorstruktur umgeben ist;
- Figur 21: das Blockschaltbild einer Auswerteeinrichtung des Röntgen-Tomographiegerätes;
- Figur 22: eine Darstellung eines Röntgen-Tomographiegerätes, welches eine exakte radiale Durchstrahlung erlaubt und bei dem die Targetstruktur und die erste Röntgenlicht-Beugungsstruktur in einem Bauteil vereint sind, wobei zusätzlich eine umlaufende Blendeneinrichtung vorgesehen ist;
- Figur 23: eine schematische Ansicht einer in der Breite verstellbaren umlaufenden Blende zur seitlichen Begrenzung eines Röntgenstrahles in einer Untersuchungsebene;
- Figur 24: eine Darstellung eines Röntgen-Tomographiegerätes mit doppelter Durchstrahlung des Austrittsfensters und zusätzlicher Durchstrahlung des Targets und der Gehäusewand, was ebenfalls eine exakte radiale Durchstrahlung erlaubt;
- Figur 25: eine Darstellung einer Blende, die den umlaufenden Röntgenfächer durch eine Taumelbewegung in seiner Höhe begrenzt und so die Bestrahlungsdosis reduziert; und
- Figur 26: einen Axialschnitt durch ein Phasenkontrast-Röntgen-Tomographiegerät mit einem Gitter, das den Untersuchungsbereich umgibt und dabei zugleich die Funktion eines Kohärenzgitters und eines Phasengitters übernimmt.

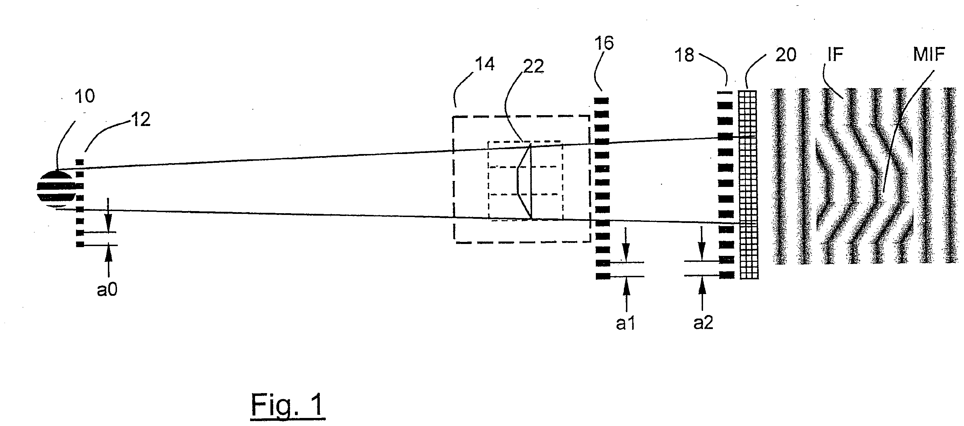

Figur 1 zeigt den Prinzipaufbau eines Phasenkontrast-Röntgen-Tomographiegerätes. Bei 10 ist eine Röntgenquelle gezeigt, die Röntgenlicht unterschiedlicher Wellenlänge bereitstellt, welches nicht kohärent ist. Hinter der Röntgenquelle 10 ist ein Kohärenzgitter 12 angeordnet, welches aus dem Licht der Röntgenquelle 10 quasi kohärentes Röntgenlicht abspaltet.

Ein solches Kohärenzgitter ist nicht notwendig, wenn die Röntgenquelle eine sehr kleinen, an die Talbot-Bedingung angepassten Emissionspunkt oder eine entsprechende Streifenstruktur ao besitzt.

Durch ein gestrichelt dargestelltes Quadrat ist ein Untersuchungsbereich 14 begrenzt.

Hinter dem Untersuchungsbereich 14 steht ein Phasengitter 16, und im Talbot-Abstand von dem letzteren ist ein Amplitudengitter 18 vorgesehen. Bei all den vorgenannten Gittern handelt es sich um Strukturen, welche für Röntgenstrahlen wirksam sind und der Talbot-Bedingung genügen.

Hinter dem Amplitudengitter 18 ist ein ringförmiger Detektorstreifen 20 angeordnet, dessen Längsrichtung mit der Haupterstreckungsrichtung des Amplitudengitters 18 zusammenfällt. Bei den dargestellten Gittern sind die einzelnen Striche senkrecht zur Zeichenebene im kleinen Abstand aufeinanderfolgend zu denken. Typische Strichabstände liegen unter 10 µm, vorzugsweise im Bereich von etwa 1 bis etwa 3 µm.

Eine Anordnung, wie sie in Figur 1 gezeigt ist, erzeugt in der Ebene des Detektorstreifens 20 ein Interferenzmuster mit hellen und dunklen Streifenabschnitten.

Das bei leerem Untersuchungsbereich 14 erhaltene Streifenmuster ist als Referenz-Interferenzmuster für den leeren Untersuchungsbereich zu verstehen.

Stellt man im Untersuchungsbereich ein Phasenobjekt 22 auf, so ändert sich das Interferenzmuster. Die Unterschiede in dem hellen und dunklen Streifen können mit dem Detektorstreifen 20 in elektrische Signale umgesetzt werden, und diese können dann in ein Phasenbild umgerechnet werden. In Figur 1 ist dabei die Randkontur des Phasenobjektes 22 in die Zeichenebene gekippt gestrichelt angedeutet. Auch das Interferenzmuster IF ist zur Veranschaulichung in die Zeichenebene gedreht.

Typischerweise erhält man das dargestellte Streifenmuster von einer Platte mit trapezförmigem Querschnitt, wie in Figur 1 gezeigt. Durch Vergleich mit dem Referenzbild erkennt man nun das für Röntgenstrahlen an sich durchlässige Phasenobjekt 22.

Wird das Phasenobjekt 22 aus dem Strahlengang genommen, laufen die Interferenzstreifen, welche in der Umgebung des Phasenbildes gefunden werden, auch in demjenigen Bereich glatt durch, in dem sich zuvor das Bild des Phasenobjektes 22 befand.

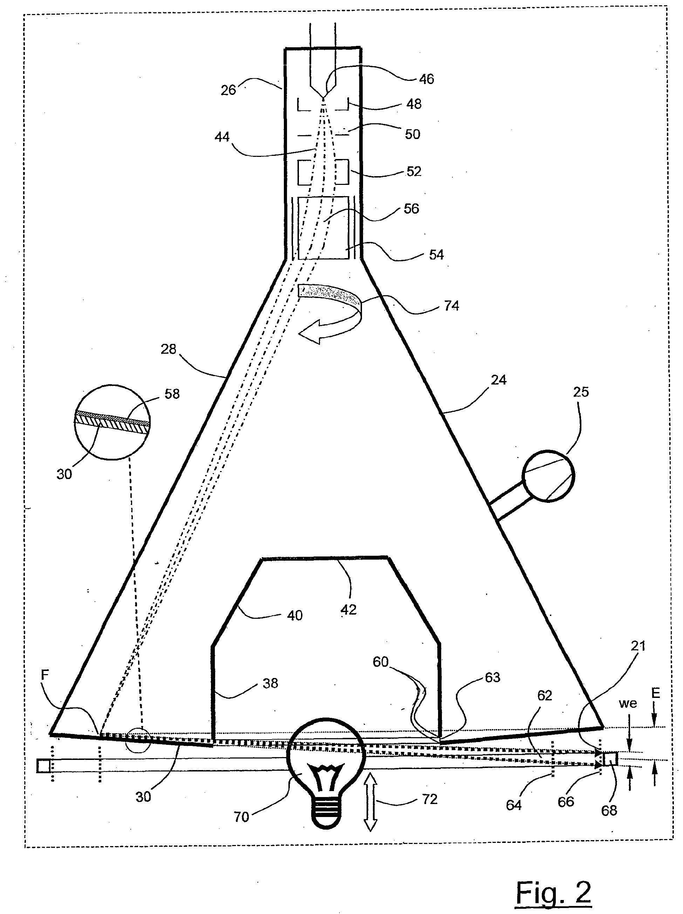

Figur 2 zeigt ein Tomographiegerät mit einem umlaufenden Röntgenemissionspunkt F. Ein Targetring 58 hat die Form eines Kegelstumpfes mit auf der Geräteachse liegender nach unten weisender Spitze.

Das Röntgentomographiegerät hat ein insgesamt mit 24 bezeichnetes gasdichtes evakuiertes Gehäuse, zu welchem ein Gehäusehals 26, eine äußere kegelförmige Gehäusewand 28, eine schräg nach unten und innen abfallende Bodenwand 30, eine axial wieder zurückführende zylindrische Gehäusewand 38, eine sich hieran anschließende nach oben zusammenlaufende kegelstumpfförmige Gehäusewand 40 und eine Endwand 42 gehören.

Der gesamte Innenraum des Gehäuses 24 ist auf einen Druck von weniger als 10⁻⁵ mbar evakuiert. Dieses Vakuum kann sowohl durch hermetische Dichtheit als auch durch gezieltes Pumpen mit einer angebauten Vakuumpumpe 25 während des Betriebes hergestellt werden.

Im Inneren des Gehäuses 24 befindet sich eine Elektronenkanone 44. Zu dieser gehören schematisch dargestellt eine geheizte Kathode 46, eine Steuerelektrode 48, eine Beschleunigungselektrode 50, eine elektrostatische Linse 52 und eine Ablenkspule 54. Alternativ kann auch eine elektrostatische Ablenkeinheit verwendet werden, die einen Ablenkplattenkondensator umfasst.

Ein Elektronenstrahl 56 ist durch seine Mittenlinie und seine Einhüllenden gestrichelt dargestellt.

Auf der Bodenwand 30 ist ein ringförmiges Target 58 angeordnet, welches aus für Röntgen-Targets üblichem Material hergestellt sein kann. Das Target 58 hat die Form eines weit geöffneten Kegelstumpfes mit einem Öffnungswinkel der Kegelspitze von etwa 160°.

An der Stoßstelle zwischen der Bodenwand 30 und der Gehäusewand 38 befindet sich ein kleiner Zwischenraum 60, der durch ein Röntgenfenster 63 verschlossen ist. Dieses erstreckt sich über volle 360°, so dass Röntgenlicht 62, welches beim Auftreffen des Elektronenstrahles 56 auf das Target 58 entsteht, in beliebiger durch die Ablenkspule 54 bzw. einen Ablenkkondensator erzeugter Winkelstellung des Fokuspunktes F bezüglich der Achse des Gehäuses 24 durch einen entsprechenden Bereich des Röntgenfensters 63 austreten kann.

Das Röntgenlicht 62 ist nur in seinem nutzbaren fächerförmigen Bereich wiedergegeben, der durch die Höhe des Röntgenfensters 63 vorgegeben ist. Vom Auftreffpunkt des Elektronenstrahles 56 auf das Target 58 gehen natürlich noch weitere Röntgenstrahlen in den oberen Halbraum aus, welche aber durch die Wände des Gehäuses 24 absorbiert werden und nicht zu Messzwecken verwendet werden. Das Gehäuse 24 ist hierzu gegebenenfalls mit zusätzlichem Abschirmmaterial zu umgeben.

Das Röntgenfenster 63 kann zugleich aus einem Material gewählt sein, welches nicht gewünschte Teile des Röntgenspektrums absorbiert. Es sind dies insbesondere langwellige Anteile des kontinuierlichen Anteils des Spektrums. Gewünscht wird ein möglichst schmalbandiges Spektrum des Röntgenlichtes, dessen mittlere Wellenlänge zusammen mit den verstellbaren Röntgenbeugungsstrukturen, hier ein ringförmiges Phasengitter 64 und ein ebenfalls ringförmiges Amplitudengitter 66, die Talbotbedingung erfüllt.

Durch die Kegelform des Targets 58 und dadurch, dass der Auftreffpunkt des Elektronenstrahles 56 auf das Target 58 in Figur 2 höher liegt als das Röntgenfenster 63 wird erreicht, dass der für Messzwecke verwendete Röntgenfächer schräg nach unten gerichtet ist und auf der gegenüberliegenden in Figur 2 rechts gelegenen Seite der Bodenwand 30 unter dieser hindurchlaufen kann.

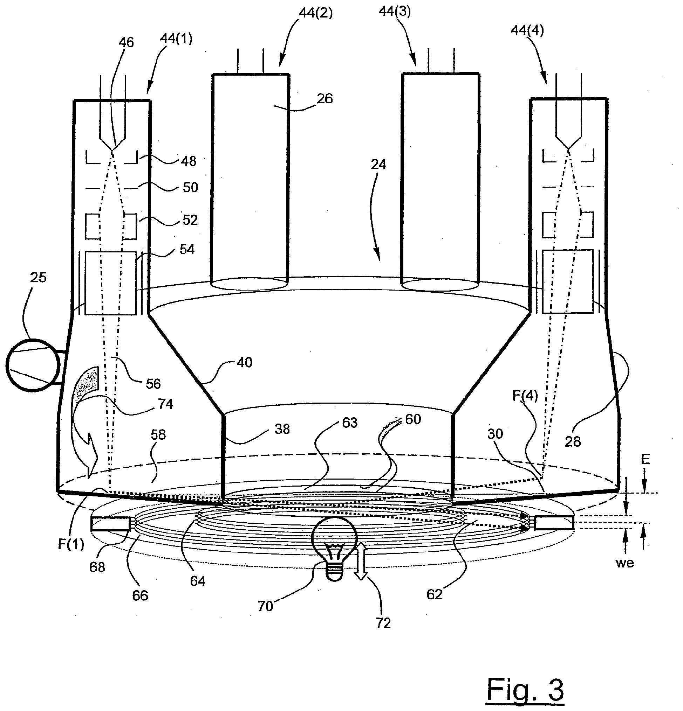

Soll das Röntgenfenster 63 etwas höher liegen als in den Figuren 2 und 3 gezeigt, kann die Bodenwand 30 an ihrem radial innen liegenden Ende eine niedere hochgekantete Umfangswand aufweisen.

Unterhalb der Bodenwand 30 ist konzentrisch zur Ge- häuseachse ein ringförmiges Phasengitter 64 angeordnet, welches gestrichelt angedeutet ist. Radial außerhalb des Phasengitters liegt koaxial ein ebenfalls ringförmiges Amplitudengitter 66. Und um das letzte herum ist ein ringförmiger Detektorstreifen 68 angeordnet.

Dieser umfasst in Streifenlängsrichtung unter sehr geringem Abstand aufeinanderfolgende Detektorelemente, deren Abstand kleiner ist als die Periode von Interferenzmustern, welche durch das Phasengitter 64 und das Amplitudengitter 66 im Bereich des Detektorstreifens 68 erzeugt werden. In Drehrichtung (axialer Richtung des Geräts) umfasst der Detektorstreifen 68 eine kleinere Anzahl (z.B. 8, 16 oder 32) unter gleichem kleinem Abstand aufeinanderfolgende Detektorelemente. Der Detektorstreifen 68 bildet somit insgesamt eine ringförmige Detektorfläche mit mehreren 100 000 Detektorpixeln in einer Ringzeile, die zum Beispiel etwa 250 000 in Umfangsrichtung aufeinanderfolgender Detektorelemente (für ein Ganzkörper-Phasenkontrast-Tomographiegerät mit etwa 80 cm lichter Weite) umfassen kann, wenn man für jeden Strich der Röntgengitter ein Detektorelement vorsieht. 8, 16 oder 32 derartiger Linien von Detektorelementen sind axial unter dem Abstand der Detektorelemente hintereinander gestapelt. Wie viele Zeilen man in axialer Richtung hintereinander vorsieht, ist eine Frage der Optimierung der Detektoren.

Prinzipiell genügt eine Detektorringzeile. Stehen Detektoren mit Pixelgrößen eines Bruchteils der gewünschten räumlichen Auflösung zur Verfügung, kann man die Phasenmessung auf mehrere Pixel, z.B. 3x3 oder 4x4 Pixel je Auflösungspunkt ausdehnen. Ist die geometrische Auflösung der gesamten Anordnung ohnehin durch andere Gegebenheiten begrenzt (z.B. Brennfleckgröße und Vergrößerung), empfiehlt sich ein 3-bis 4-zeiliger Detektor je Auflösungszeile. Eine weitere Erhöhung der Zeilenzahl ist wegen der besseren Nutzung der Röntgenstrahlung sinnvoll, aber nur möglich, wenn die Gitterlamellen mit zunehmender axialer Entfernung von der Bahnebene des Brennpunktes F entsprechend schräg gestellt werden, was den Aufwand für die Gitteranordnung wiederum erhöht.

Wie später noch genauer beschrieben wird, ist es für das Ausmessen der Interferenzfiguren an sich günstig, wenn die Abstände der Detektorelemente möglichst klein sind. Man erhält dann pro Streifenmuster-Periode eine größere Anzahl von Stützstellen, aus denen sich der Verlauf der Sinusform bei der Intensitätsverteilung direkt ausrechnen lässt.

Hat man eine geringere Anzahl von Stützpunkten, möglicherweise nur ein Detektorelement pro Auflösungszelle oder weniger Detektorelemente als Auflösungszellen, muss man die Phasenmessung der Wellenfront dadurch ermöglichen, dass man eine derjenigen Strukturen, welche für die Erzeugung des Interferenzbildes verantwortlich sind (Röntgenquelle, Kohärenzgitter, Phasengitter, Amplitudengitter) um sehr kleine Abstände verschiebt, die Bruchteile des Abstandes zweier Gitterlinien betragen, um bei bekannten kleinen Verschiebungen eines beugungsrelevanten Bauteiles zusätzliche Informationen über den Intensitätsverlauf zu erhalten, worauf man dann diese zusätzlichen Messpunkte als zusätzliche Stützpunkte zum Anpassen einer Sinuskurve und zur Bestimmung der Phasenlage dieser Sinuskurve verwenden kann.

Dies wird weiter unten noch genauer beschrieben. Hier sei zunächst angenommen, dass die Teilung des Detektorstreifens 68 ausreichend klein ist, um ohne die oben angesprochenen Relativbewegungen um sehr kleine Strecken das Interferenzmuster ausreichend präzise ausmessen zu können, um hieraus die Phasenlage der Wellenfront zu bestimmen.

Die Röntgengitter, d.h. das Phasengitter 64 und das Amplitudengitter 66 sind in der Zeichnung als Gitter dargestellt, welche in Richtung der Geräteachse (oder bei besonders breiten Streifen sogar in Richtung der gegenüberliegenden Emissionspunktbahn orientierte) abwechselnd aufeinanderfolgende Schichten aus röntgendurchlässigem Material und Röntgenstrahlen absorbierendem Material aufweisen.

Wichtig für die Zwecke der Erfindung ist nur, dass die beiden Röntgengitter zusammen ein Interferenzbild herstellen. Ein solches Interferenzbild kann man auch mit Röntgengittern erhalten, bei denen die abwechselnd aufeinanderfolgenden Schichten aus durchlässigem und absorbierendem Material in Umfangsrichtung periodisch hintereinander gestapelt sind.

Wie aus der Zeichnung ersichtlich und aus der Geometrie des Röntgenfensters 63 und dem Auftreffpunkt des Elektronenstrahles 56 auf das Target 58 ableitbar, hat das Röntgenlicht 62 die Form eines sehr schwach divergierenden, sehr flachen, fächerförmigen Röntgenbündels.

Der fächerförmige Röntgenstrahl 62 zeichnet sich bezüglich seines Verlaufs und seiner Geometrie durch folgende Winkel aus: Einen Winkel E, unter welchem seine Umlaufebene zu einer Ebene geneigt ist, die transversal auf der Geräteachse steht. Ferner einen Elevations-Fächerwinkel we, der den Öffnungswinkel des Fächers in einer axialen Schnittebene darstellt. Ferner einen Öffnungswinkel des Fächers in Umfangsrichtung wu, der den Öffnungswinkel des Fächers in der Umlaufebene darstellt. Diese verschiedenen Winkel sind in die Figuren 2 bis 4 eingetragen. In den anderen Figuren sind sie nur teilweise eingezeichnet. In der Umgebung der Achse des Gehäuses 24 ist jeweils ein Untersuchungsobjekt 70 (beispielhaft in Form einer Glühbirne) mit seiner bevorzugten Bewegungsrichtung 72 gezeigt.

Der Röntgenstrahl 62 durchsetzt bei vorgegebener Stellung des Untersuchungsobjektes 70 letzeres in genau einer Schnittebene je Detektorauflösungszeile. Um andere Schichten des Untersuchungsobjektes 70 untersuchen zu können, kann letzteres durch einen schematisch angedeuteten Antrieb 72 bevorzugt in axialer Richtung verfahren werden. Alternativ kann man das Röntgen-Tomographiegerät verfahren.

Aus der obigen Beschreibung des Gerätes nach Figur 2 ist ersichtlich, dass durch entsprechende Bestromung der Ablenkspule 54 der Elektronenstrahl auf einer Kreisbahn um die Achse des Gehäuses 24 bewegt werden kann, wie in der Zeichnung durch einen Pfeil 74 angedeutet. Bei dieser Bewegung wandert dann der Auftreffpunkt F des Elektronenstrahles 56 auf dem Target 58 auf einen zur Gehäuseachse konzentrischen Kreis, und das Röntgenlicht 62 tritt dann in entsprechender Winkelausrichtung aus dem Röntgenfenster 60 aus.

Figur 3 zeigt ein abgewandeltes Tomographiegerät. Komponenten, die funktionell unter Bezugnahme auf Figur 2 schon beschriebenen Komponenten entsprechen, sind wieder mit denselben Bezugszeichen versehen und brauchen nicht nochmals im Einzelnen beschrieben zu werden.

Bei dem Tomographiegerät nach Figur 3 sind mehrere Elektronenkanonen 44 vorgesehen, die ähnlichen Aufbau aufweisen wie die in Figur 2 gezeigte Elektronenkanone. Die Elektronenkanonen 44 gemäß Figur 3 sind nun aber unter regelmäßigem Abstand über die Umfangserstreckung des Gehäuses 24 verteilt, so dass die von ihnen jeweils erzeugten Elektronenstrahlen 56 nur einen Teilbereich der Umfangserstreckung des ringförmigen Targets 58 überstreichen müssen. Durch sequenzielle Ansteuerung der verschiedenen Elektronenkanonen 44 wird dann insgesamt die gesamte Umfangserstreckung des Targets 58 abgefahren, wobei auch mehrere Elektronenkanonen gleichzeitig benutzt werden können, sofern sich ihre Röntgenstrahlfächer auf den Detektoren nicht überdecken.

Bei der Anordnung nach Figur 3 brauchen die verschiedenen Elektronenkanonen nicht in Umfangsrichtung aufeinanderfolgend aktiviert zu werden. Man kann die Elektronenkanonen auch in anderer Reihenfolge aktivieren, was im Hinblick auf Wärmeprobleme im Target 58 von Vorteil sein kann. Vorteilhaft, aber nicht zwingend notwendig ist nur, dass in einem vorgegebenen Zeitraum (Scanzyklus) alle Elektronenkanonen 44 genau ein Mal so aktiviert wurden und dass ihr Elektronenstrahl 56 auf dem zugeordneten bogenförmigen Segment der gesamten Umfangserstreckung des Targets 58 bewegt wurde.

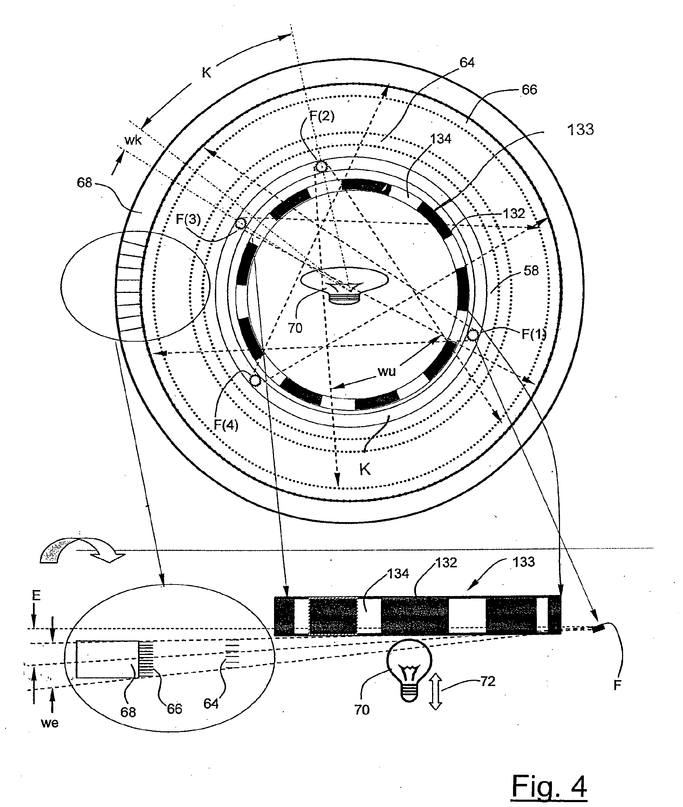

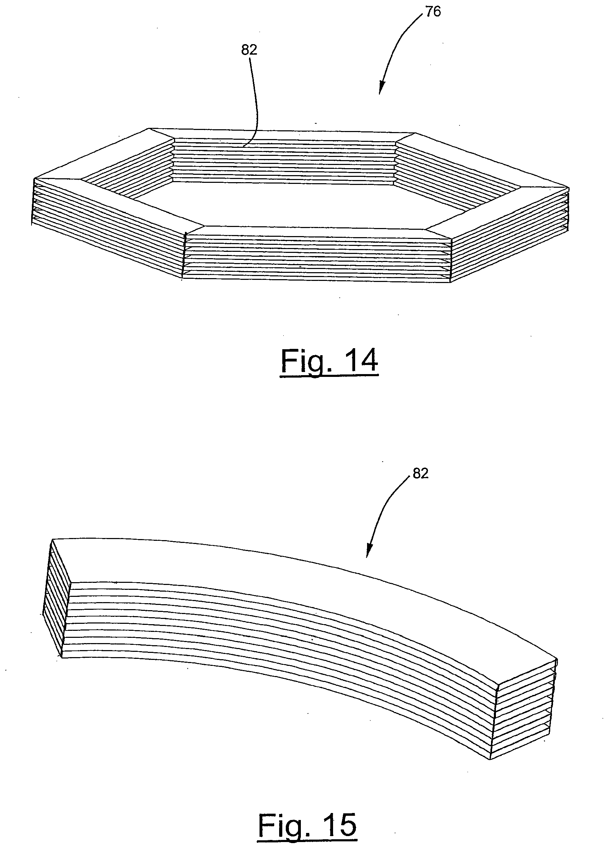

Figur 4 zeigt eine Aufsicht auf den unteren Abschnitt des Tomographiegerätes, wobei die Winkelerstreckung des Röntgenfächers in zur Geräteachse senkrechter Richtung (Untersuchungsebene) und die Auffächerung des Röntgenlichtes in vertikaler Richtung (Elevationsauffächerung) genauer dargestellt sind. Ferner sind in der Zeichnung für das Phasengitter 64 und das Amplitudengitter 66 einige Striche in der Ausschnittsdarstellung angedeutet. Der Aufbau dieser ringförmigen Röntgengitter wird später unter Bezugnahme auf die Figuren 14 bis 22 noch genauer erläutert. Mit F ist in Figur 4 der Auftreffpunkt des Elektronenstrahles 68 auf das Target 58 bezeichnet. K steht für die Spur, auf welcher sich der Brennfleck F des Elektronenstrahls über das Target 58 bewegt.

Mit 133 ist in Figur 4 eine segmentierte Blende bezeichnet, welche als Blendengitter ausgebildet ist. Sie umfasst eine große Anzahl in Umfangsrichtung unter kleinem Abstand aufeinanderfolgender Blendenstäbe 132, zwischen denen Blendenfenster 134 verbleiben.

Die Blende 133 wird raumfest unter kleinem Abstand auf der Innen- oder Außenseite des Röntgenfenster 63 angeordnet und der Elektronenstrahl 56 wird durch geeignete Erregung der Ablenkspule 54 in Inkrementen auf dem Target 58 derart versetzt, dass der Fokus F jeweils radial mit der Mitte eines der Blendenfenster 134 fluchtet.

Man hat somit für jeden gegebenen Augenblick in der Untersuchungsebene einen Röntgenstrahl, dessen Breite durch die Weite des Blendenfensters 134 und den Bahnradius des Elektronenstrahlauftreffpunktes F vorgegeben ist.

Auch eine gezielte exzentrische Lage des Untersuchungsvolumens kann durch Umlaufwinkel-abhängige Versetzung des Elektronenstrahlauftreffpunktes F zur Mitte der Blendenfenster 134 erreicht werden.

Die Auswertung der Ausgangssignale des Detektorstreifens 68 erfolgt bei Verwendung der Blendenkörper nach Figur 3 und nach Figur 4 so, dass man jeweils einen Teilbereich des Detektorstreifens 68 scharf schaltet bzw. dessen Ausgangssignale auswertet, auf welchem nutzbare Interferenzmuster erhalten werden. Das Auswählen dieses Bereiches kann auch unter Auswertung der Ablenksignale für die Ablenkspule 54 erfolgen.

Durch enge seitliche (Umfangswinkel-) Begrenzung des Röntgenstrahles in der Untersuchungsebene wird die Dosis für den Patienten reduziert, und insbesondere bei einer Ausführung gemäß Figur 3 wird die Bildaufnahmegeschwindigkeit erhöht.

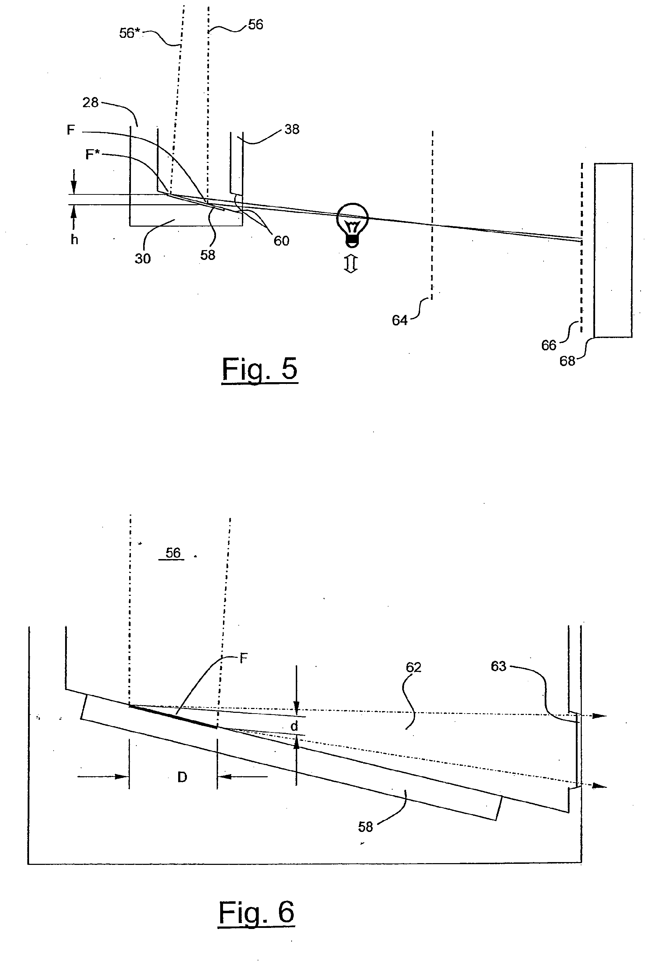

Die Figuren 5 und 6 zeigen noch einmal schematisch den Strahlengang zwischen dem ringförmigen Zwischenraum 60 und dem Detektorstreifen 68 in axial vergrößertem Maßstab.

Aus diesen Figuren ist auch gut ersichtlich, dass durch die Schrägstellung des Targets 58 eine Reduzierung der gesehenen Größe der punktförmigen Röntgenlichtquelle erhalten wird, welche durch den Fokus F des Elektronenstrahles 56 auf dem Target 58 gebildet ist. Hat der Querschnitt des einfallenden Elektronenstrahls 56 die Größe D, erscheint in axialer Richtung gesehen von dieser Größe nur noch ein Bruchteil d.

Auf diese Weise ist es möglich, ein Objekt in sehr dünnen Schichten zu untersuchen und gleichzeitig die räumliche Kohärenz in axialer Richtung so zu verbessern, dass gegebenenfalls auf das in Figur 1 dargestellte Kohärenzgitter 12 verzichtet werden kann und trotzdem die Talbotbedingung erfüllt ist.

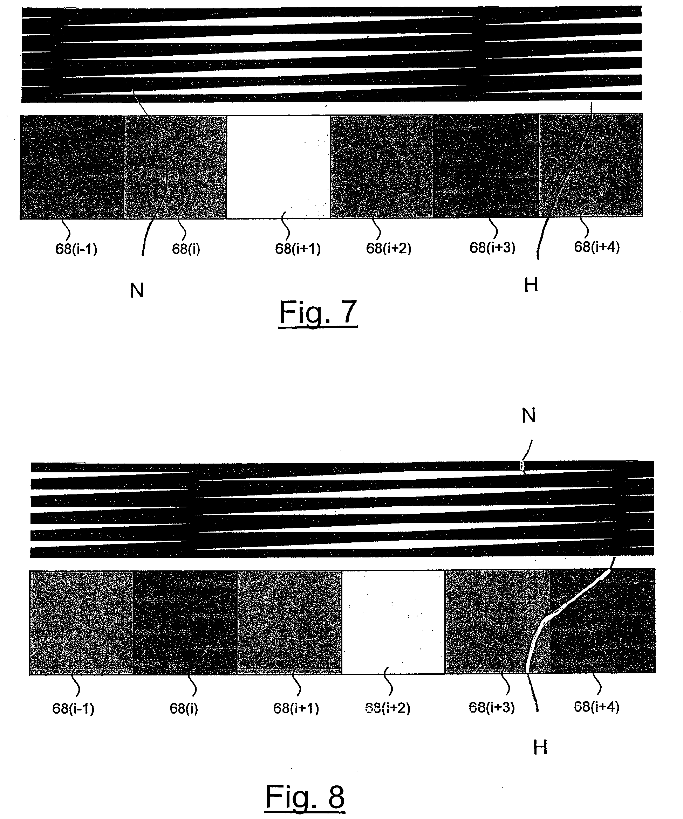

Figur 7 zeigt schematisch ein Interferenzmuster, wie es in der lichtempfindlichen Fläche des Detektorstreifens 68 für eine vorgegebene Relativstellung von Phasengitter 64 und Amplitudengitter 66 erhalten wird. Unter Relativstellung ist hier sowohl der Abstand senkrecht zur Gitterfläche als auch die Neigung der Gitterlinien der beiden Gitter gegeneinander zu verstehen.

Das Interferenzmuster umfasst beim dargestellten Ausführungsbeispiel horizontale Streifen H, welche unter gleichem Abstand nebeneinander liegen. Eine zweite Streifenschar, welche unter kleinem Winkel geneigt zu dem Streifen H verläuft, ist mit N bezeichnet. Die Streifen H und N bilden in Figur 7 additiv übereinandergelegt gesehen Bereiche mit großem Schwarzanteil und Bereiche hoher Helligkeit, die jeweils grob gesprochen rautenähnliche Struktur haben.

Unterhalb des Streifenmusters sind als Grauwertbild die zugehörigen Helligkeitswerte von Detektorelementen 68i, 68i+1 bis 68i+6 dargestellt.

Der Helligkeitsverlauf von links nach rechts entspricht einem sinusförmigen Verlauf mit einem Grundwert, da keine negative Helligkeit vorkommen kann. Man kann nun durch mathematische Verfahren (Anpassen, Fourier-Transformation) die Amplitude und Phasenlage in diesen sechs Detektorelementen berechnen.

Figur 8 zeigt ein ähnliches Bild, wobei aber die Phasenlage zwischen den Interferenzen, welche durch Phasengitter und Amplitudengitter erhalten werden, geändert ist. Eine derartige Änderung der Phasenlage kann erhalten werden durch ein Verschieben des Objektes und/oder ein Verschieben der Röntgenlichtquelle und/oder ein Verschieben des Phasengitters und/oder ein Verschieben des Amplitudengitters und/oder durch ein Verkippen des Phasengitters und/oder ein Verkippen des Amplitudengitters.

Man erkennt, dass sich das geänderte Interferenzmuster auch in einer geänderten Helligkeitsverteilung im unter dem Interferenzmuster wiedergegebenen Helligkeitsprofil ergibt, und zwar in der Form, dass die sinusartige Helligkeitsverteilung sich um ein Detektorpixel nach rechts verschoben hat.

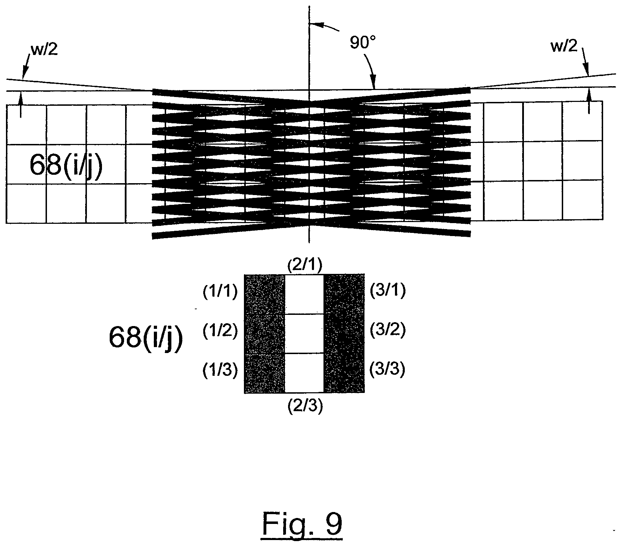

Betrachtet man jetzt alle sechs Pixel 68i bis 68-i+4, die zu einem Auflösungspixel gehören, kann man auf eine veränderte Phasenlage, die z.B. auch durch ein Objekt im Röntgenstrahl hervorgerufen wird, zurückrechnen. Verwendet man eine Detektoranordnung mit nur einer Zeile von Detektorpixeln, hat man ein sehr unförmiges, längliches Auflösungspixel in Form von 6x1 Detektorpixeln. In den Figuren 9 bis 11 ist deshalb veranschaulicht, wie man z.B. aus 4x4 oder 3x3 Detektorpixeln die Phasenmessung eines eher quadratischen Auflösungspixels durchführen kann.

Figur 9 zeigt eine abgewandelte Geometrie, bei welcher das Phasengitter und das Amplitudengitter um entgegengesetzt gleich große Winkel gegen eine Referenzrichtung verkippt sind. Diese Anordnung wäre ebenfalls nur für ein längliches Auflösungspixel geeignet, da die obere und untere Pixelreihe mit der mittleren identisch ist und die Phasenlage der Sinusschwingung eigentlich nur aus drei Stützstellen bestimmt werden kann.

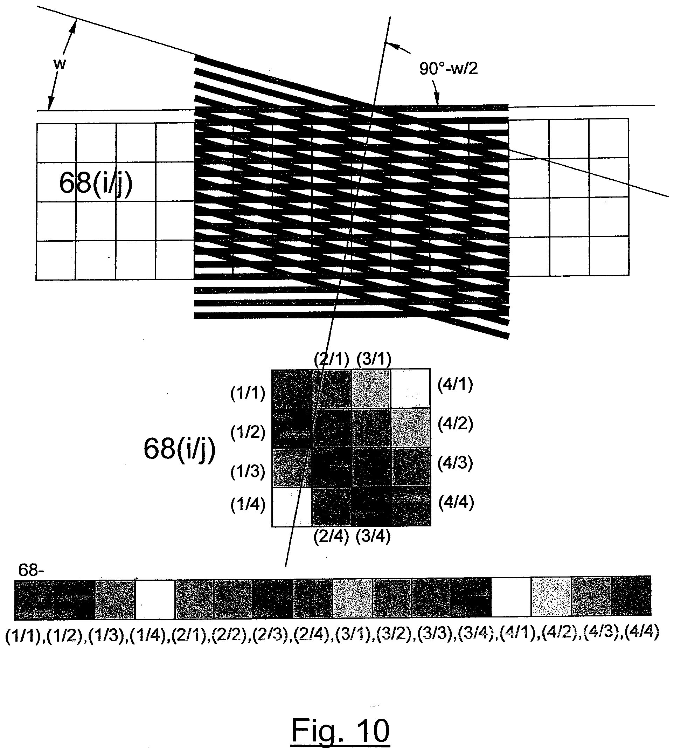

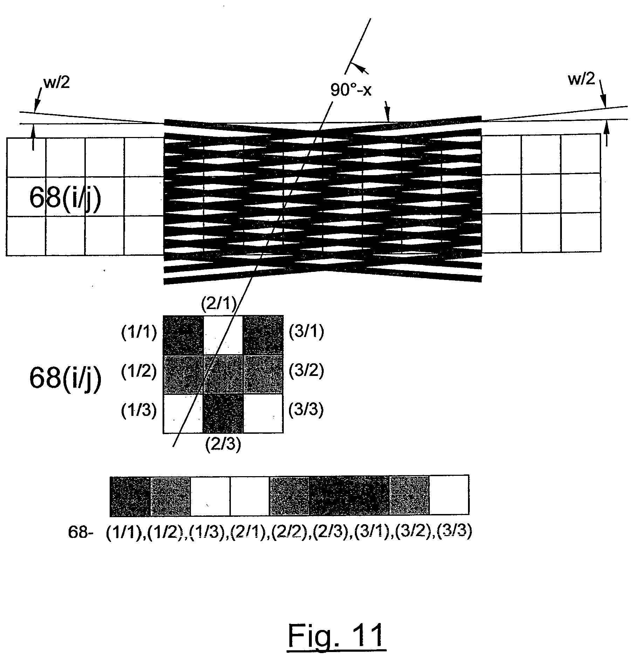

Gemäß dem Abtasttheorem wäre dies zwar ausreichend, in der Praxis sind solche Röntgenbilder jedoch auch noch durch Rauschen verfälscht, weshalb es besser wäre, mehr Abtastpunkte für die Ermittlung der Phasenlage auszuwerten. Durch geeignete Schrägstellung der Gitter gemäß den Figuren 10 und 11 sind nun alle 16 bzw. 9 Pixel des 4x4 bzw. 3x3 Pixelgrößen Auflösungspixel in der Sinusperiode ungefähr gleich verteilt.

In der Reihenfolge (1/1), (1/2), (1/3), (1/4), (2/1), (2/ 2), (2/3), (2/4), (3/1), (3/2), (3/3) usw. ergibt sich eine sinusartige Intensitätsverteilung, aus der wie in Figur 7 und 8 die Phasenlage und Amplitude bestimmt werden kann. Man erkennt, dass man durch die Verkippung der Gitter ein Interferenzmuster erhält, welches in horizontaler Richtung kleinere Periodenlänge aufweist. Die Periodenlänge ist also durch den Verkippwinkel w einstellbar. Figur 11 zeigt ein Streifenmuster, welches dadurch erhalten wird, dass man für Phasengitter und Amplitudengitter geringfügig unterschiedliche Gitterkonstanten wählt. Im Übrigen entspricht die Anordnung der Gitter derjenigen nach Figur 9.

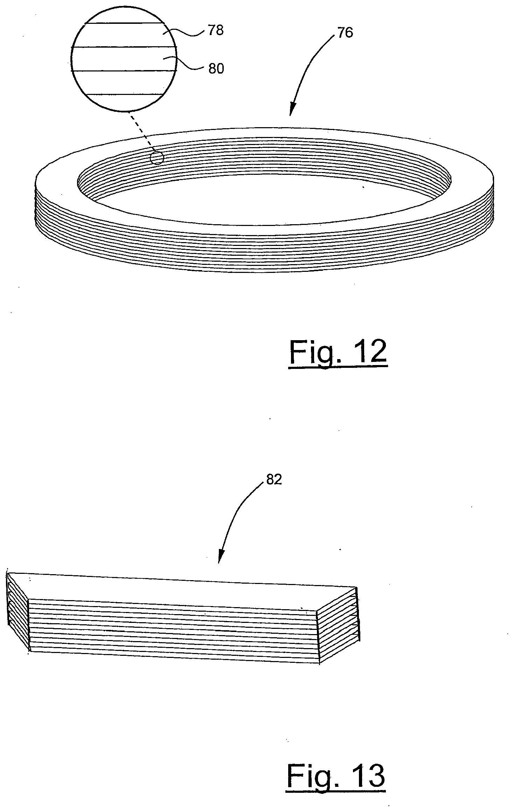

Man erkennt, dass auf diese Weise das Streifenmuster gegenüber dem Streifenmuster nach Figur 9 in der horizontalen Richtung "geschert" ist, es ist also entsprechend dem Streifenmuster in Figur 10 gleichwertig auswertbar. Durch geeignete Wahl der Kippwinkel der Gitter und der Gitterkonstanten ist es also möglich, das Interferenzmuster jedes Auflösungspixels so einzustellen, dass in jedem fast beliebig gewählten zugehörigen Detektorpixelarray (z.B. 3x4) eine Sinusschwingung mit allen zugehörigen Detektorpixeln gut angepasst werden kann. Durch gezielt verschiedene Gitterkonstanten ist es sogar möglich, ohne Nachteile eines der Gitter absolut horizontal anzuordnen, wie in den Figuren 12 bis 16 gezeigt.

In Figur 12 ist ein ringförmiges Röntgengitter insgesamt mit 76 bezeichnet. Es besteht aus einer axialen Aufeinanderfolge von Schichten 78, 80, die aus unterschiedlichem Material bestehen. Der Unterschied in den Materialien kann in der Ausbreitungsgeschwindigkeit von Röntgenlicht liegen, dann handelt es sich bei dem Röntgengitter 76 um ein Phasengitter. Der Unterschied in den Schichten 78, 80 kann aber auch in der Absorption von Röntgenlicht liegen, dann stellt das Röntgengitter 76 ein Amplitudengitter dar.

Typischerweise werden Röntgengitter so hergestellt, dass man eine für Röntgenlicht transparente Grundstruktur hat, welche mit einem feinen Nutenmuster versehen ist. Diese Grundstruktur wird dann mit einem anderen Material dünn überschichtet. Dieses dünne Material beeinflusst beim Nutgrund, der senkrecht auf der Fortpflanzungsrichtung des Röntgenlichtes steht, das Röntgenlicht nur geringfügig, da die Schicht dort sehr dünn ist. In den Bereichen der Nutwände muss das Röntgenlicht dagegen das aufgedampfte Material auf langem Weg durchsetzen, so dass man eine große Beeinflussung erhält.

Das Röntgengitter hat somit breite Bereiche, in denen das Licht phasenmäßig und amplitudenmäßig nur wenig beeinflusst wird, und schmale Bereiche, welche das Licht stark abschatten, bzw. eine starke Phasenänderung bewerkstelligen.

Das ringförmige Röntgengitter 76 gemäß Figur 12 ist makroskopisch einstückig.

Man kann derartige ringförmige Röntgengitter aber auch aus mehreren Segmenten zusammensetzen.

Figur 13 zeigt ein erstes derartiges stabförmiges Ringsegment 82 mit unter 30° abgeschrägten Endflächen. Derartige Ringsegmente 82 lassen sich zu einem Sechseck-Ring zusammensetzen, so dass man ein Röntgengitter 76 erhält, wie es in Figur 14 gezeigt ist.

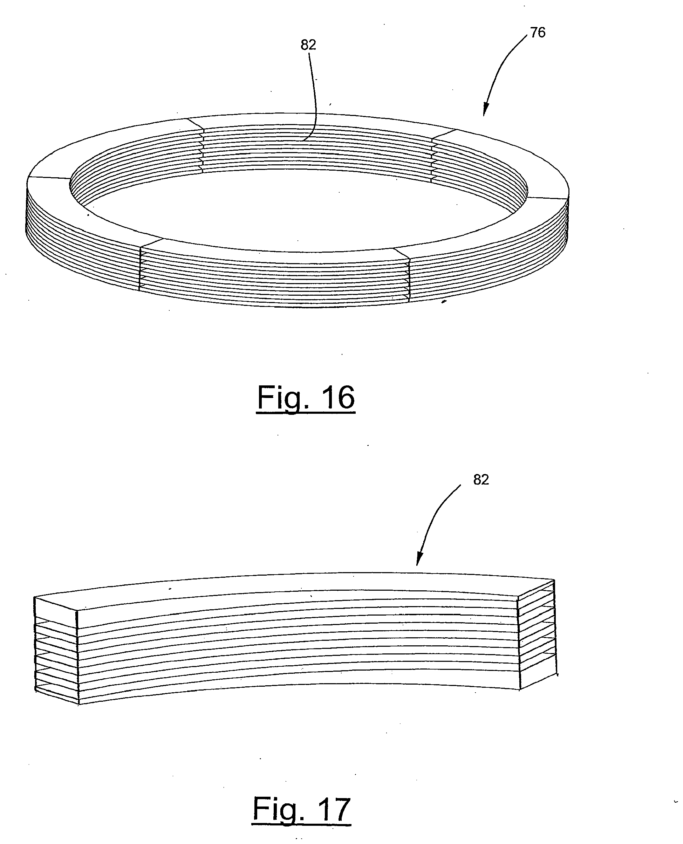

Ähnlich kann man mit kreisbogenförmigen Ringsegmenten 82 mit radialen Endflächen, wie sie in Figur 15 dargestellt sind, ein ringförmiges Röntgengitter 76 zusammensetzen, das in Figur 16 gezeigt ist.

Um ein Röntgengitter 76 zu haben, bei welchem die Gitterlinien geringfügig geneigt zu seinen Stirnflächen verlaufen, kann man ein Ringsegment 82 herstellen, wie es in Figur 17 gezeigt ist. Bei diesem sind die Gitterlinien schräg zur Ringachse geneigt.

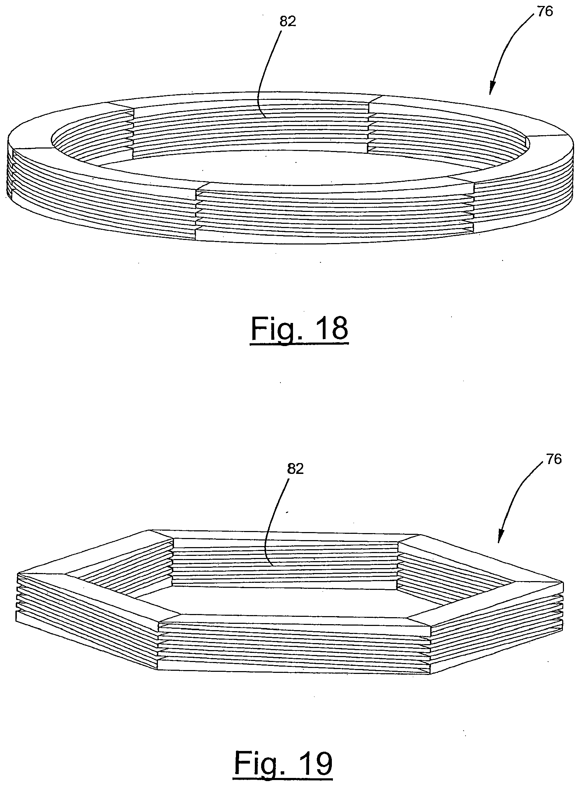

Mit Ringsegmenten 82 gemäß Figur 17 lässt sich dann ein ringförmiges Röntgengitter 76 herstellen, wie es in Figur 18 gezeigt ist. Man erkennt, dass die Schichten 78, 80 eine mehrgängige Wendel bilden.

Bei einem solchen Röntgengitter 76 kann man die außen liegenden Endflächen als Justier- und Passflächen verwenden, die mit transversal zur Geräteachse verlaufenden Schultern zusammenarbeiten. Es ist also nicht notwendig, Anlageflächen des Gehäuses unter vorgegebenem kleinem Winkel zur Geräteachse auszubilden, um so Gitterlinien zu verkippen. Man braucht auch keine Justiermittel vorzusehen, um die Neigung der Gitterlinien einzustellen.

Figur 19 zeigt ein ähnliches Röntgengitter 76 wie Figur 18, das jedoch als Sechseck-Ring ausgebildet ist.

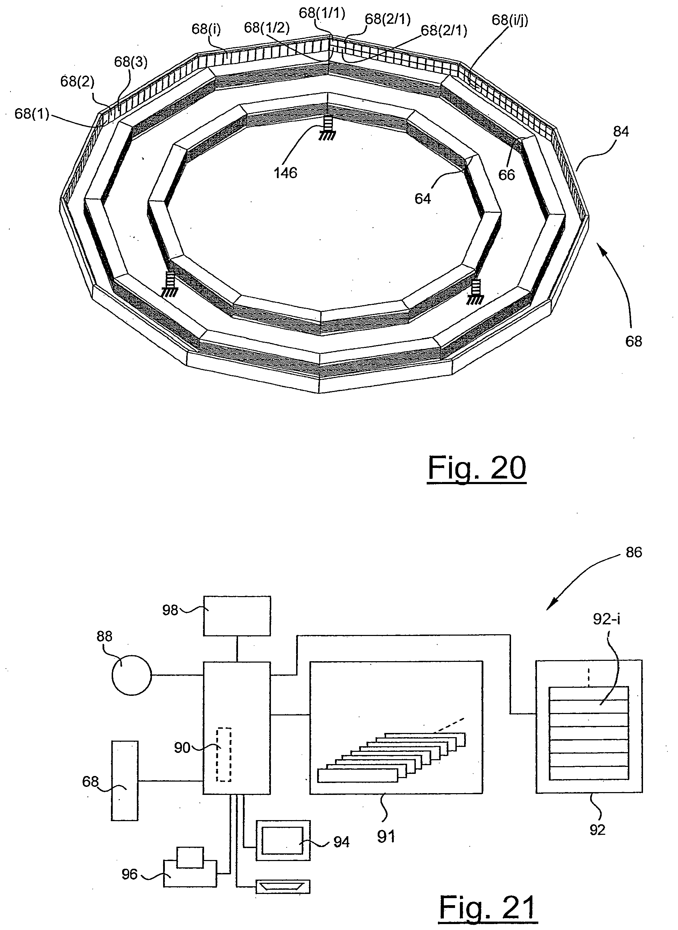

Figur 20 zeigt ein innenliegendes Phasen-Röntgengitter 64 und ein dieses unter Abstand umgebendes ringförmiges Amplituden-Röntgengitter 66, die beide ähnlich wie das Röntgengitter 76 ausgebildet sind, jedoch zwölf an den Umfangsenden entsprechend abgeschrägte Segmente aufweisen. Auf diese Weise lassen sich großen Durchmesser aufweisende Röntgengitter und Detektorzeilen bzw. -streifen aus kleineren Einheiten zusammenbauen.

Um die Ringgitter herum ist der Detektorstreifen 68 gezeigt, welcher ebenfalls aus geraden Detektorsegmenten 84 aufgebaut ist, deren Enden unter 15° abgeschrägt sind. Er hat Detektorelementzeilen 68-1, 68-2, .. 68-i, ....

Es ist leicht vorstellbar, in Figur 20 nun 3- oder höherzeilige Detektorstreifen, einen Gitterring nach Figur 19 und einen Gitterring nach Figur 14 zu kombinieren und das Auswerteverfahren nach Figur 10 anzuwenden. Höherzeilige Detektorstreifen mit Pixeln 68(1/1), ..., 68(i/j) sind hier durchaus sinnvoll und können verwendet werden, um mehrere Schichten der Tomographieaufnahme gleichzeitig zu stellen. Insbesondere haben handelsübliche Streifendetektoren auch in der kleineren Dimension meist 64 und mehr Zeilen und eine Ausdehnung von mehr als 6 mm. Bei Auflösungspixeln von beispielsweise 300 µm sind dies bereits 20 gleichzeitig gemessene Schichten. Auch für die Gitterstreifen ist eine Herstellung in einer Breite von 6 mm weit wirtschaftlicher als mit einer Breite von 0,3 mm. Auch die Montage der Gitterstreifen ist so vereinfacht.

Bei der Ausbildung zu breiter Streifen muss bei üblichen Dimensionen größer 10 mm jedoch bedacht werden, dass gegebenenfalls auch die Ausrichtung der Gitterplatten grob in Richtung der Emissionsebene zeigen muss, weil bei den üblichen Dimensionen sonst ein sogenannter Jalousieeffekt die Gitterwirkung beeinträchtigt oder gar zunichte macht. Hierzu ist dann eine zusätzliche pyramiden-, kegel- oder kugelartige Ausbildung der Gitterringe notwendig.

Auf diese Weise lassen sich großen Durchmesser aufweisende Röntgengitter und Detektorzeilen aus kleineren Einheiten zusammenbauen.

In Figur 21 ist schematisch eine Auswerteeinheit 86 dargestellt, welche die Ausgangssignale des Detektorstreifens 68 in ein Phasenbild des Objektes 70 umsetzt. Die Auswerteeinheit 86 hat einen programmierbaren Prozessor 87, der mit einem Stellungsgeber 88 zusammenarbeitet, der ein Signal bereitstellt, welches der Stellung des Elektronenstrahls 56 in Umfangsrichtung entspricht. Dieses Signal kann z.B. vom Aktivierungssignal der Ablenkspule 54 abgeleitet sein. Dieses Signal ist dann zugleich eine Information darüber, wie der fächerförmige Röntgenstrahl 62 in der Untersuchungsebene winkelmäßig ausgerichtet ist. Der Prozessor 87 hat dann eine Untereinheit 90, welche mit den Ausgangssignalen des Detektorstreifens 68 beaufschlagt ist und diese speichert.

Ist die Teilung der Detektorelemente 68(i, j) deutlich kleiner als die Teilung der Interferenzfigur, so kann die Untereinheit 90 direkt an die Ausgangssignale benachbarter Detektorelemente ein sinusförmiges Intensitätsprofil anpassen, z.B. durch Least Square Fit oder Fourier-Transformation. Dieses Intensitätsprofil wird mit einem Referenz-Intensitätsprofil verglichen, welches man ohne Objekt erhalten hatte und in einem Referenzprofilspeicher 91 abgelegt ist. Durch Vergleichen wird dann die Phasenverschiebung berechnet, welche auf das Objekt zurückzuführen ist.

Der mittlere Absorptionswert des Objektes und die Amplitude der Sinusform-ähnlichen Pixelinformation stehen, wie bei anderen Phasenkontrastverfahren, ebenfalls als bildgebende Information zur Verfügung und können in den weiteren Verarbeitungseinheiten ebenfalls verwertet werden.

Die Phasenverschiebungen für die verschiedenen Bildpixel werden dann in einem Speicher 92 in den Schichten des Objektes entsprechenden Speicherbereichen 92-1, 92-2, ... 92-i, ... abgelegt, in welchem die pixelweise berechneten Phasenverschiebungen ein zunächst numerisches Bild des Objektes darstellen.

Indem man diese Zahlenwerte ähnlich behandelt wie Grauwerte eines üblichen Bildes, kann man dann mit den herkömmlichen tomographischen Rekonstruktionsverfahren, welche selbstverständlich auch auf Teilvolumina angewandt werden können, und mit herkömmlichen Bildverarbeitungsverfahren visuell bezüglich des Kontrastes verbesserte Tomographie-Bilder erzeugen, die dann ein Beobachter auf einem Bildschirm 94 evaluiert. Alternativ oder zusätzlich werden die Bilder auch auf einem Drucker 96 ausgegeben und in einem Patienten- oder Objektdatenbank-Speicher 98 abgelegt.

Stehen keine Detektorstreifen 68 zur Verfügung, bei denen die Teilung der Detektorelemente 68-i deutlich (Faktor 4 und mehr) kleiner ist als die Teilung des Interferenzmusters, erfolgt die Bestimmung der durch das Objekt geänderten Phasenlage nacheinander dadurch, dass man Versuchsbedingungen geringfügig ändert, die einen Einfluss auf die Entstehung der Interferenzmuster haben. Entsprechende Änderungen sind in erster Linie Änderungen in der Lage des Brennfleckes F des Elektronenstrahles 56, Änderungen in der Lage von Phasengitter 64 und Amplitudengitter 66.

Diese Änderungen müssen, klein gegenüber der Gitterkonstanten des verwendeten Röntgengitters sein. Sie werden in eine der Abtastpunkteanzahl für das sinusähnliche Grauwertsignal entsprechende Schrittanzahl unterteilt, wobei die Weg-Summe der Schritte insgesamt der Gitterkonstanten des verwendeten Röntgengitters entspricht. Solche kleine Änderungen erhält man durch Radialverlagerungen des Brennflecks F auf dem möglichst flach geneigten Target 58 oder durch Verstellen oder Verkippen eines Röntgengitters durch Mikroaktoren 146, z.B. piezoelektrische Aktoren, wie sie in Figur 20 am Phasen-Röntgengitter 64 translatorisch angreifend angedeutet sind.

Es versteht sich, dass man auch mehrere der genannten Änderungen in Kombination verwenden kann.

Für verschiedene Stellungen der beweglichen Komponenten misst man dann die Interferenzfigur wieder aus. Die verschiedenen Ausgangssignalen der Detektorelemente 68-i, welche man für verschiedene Relativlagen k ermittelt, werden in Speicherfeldern des Speichers 90 abgelegt.

Dann hat man für jedes Pixel eine größere Anzahl von Stützpunkten (analoge Speicherzellen der Speicherbereiche 90-k), die für die Bestimmung einer Intensitäts-Sinuskurve verwendet werden. Dies erfolgt im Einzelnen ähnlich wie oben für den Fall sehr fein geteilter Detektorstreifen beschrieben. Aus dieser Sinuskurve wird dann wieder die Phasenverschiebung gegenüber der Referenzmessung ohne Objekt berechnet. Bei Bedarf können auch hier zusätzlich zur Phaseninformation auch der mittlere Grauwert und die Amplitude des Sinusform-ähnlichen Verlaufes weiter verwertet werden.

Aus den Phasenlagen, die man für die verschiedenen Pixel mit und ohne Objekt gemessen hat, kann man dann wieder das Phasenbild des Objektes erstellen.

Es versteht sich, dass das Refrenz-Interferenzbild des Tomographiegerätes allein (ohne Objekt; Referenzbild) eine Gerätekonstante ist, die nur in größeren Abständen neu bestimmt zu werden braucht, um Alterungseffekte und gegebenenfalls auch thermische Instabilitäten auszuschalten. Auch dann, wenn man für spezielle Untersuchungen die Geometrie der Anordnung ändert, muss natürlich das Referenz-Interferenzmuster neu bestimmt werden.

Man erkennt, dass das oben beschriebene Phasenkontrast-Tomographiegerät keine mechanisch auf hohe Genauigkeit zu bearbeitenden Komponenten aufweist mit Ausnahme der Röntgengitter und des Detektorstreifens. Lediglich das Objekt muss mechanisch in dem Phasenkontrast-Tomographiegerät bewegt werden, wenn das Aufnahmevolumen größer als die 20 Schichten umfassende Gitterstruktur sein soll.

Es eignet sich im medizinischen, dentalen und veterinärmedizinischen Bereich somit zum Einsatz auch unter Nicht-Laborbedingungen. Gleiches gilt für den Einsatz auch in den Bereichen der Materialprüfung und des Sicherheitswesens (Kontrolle von Gepäckstücken usw.).

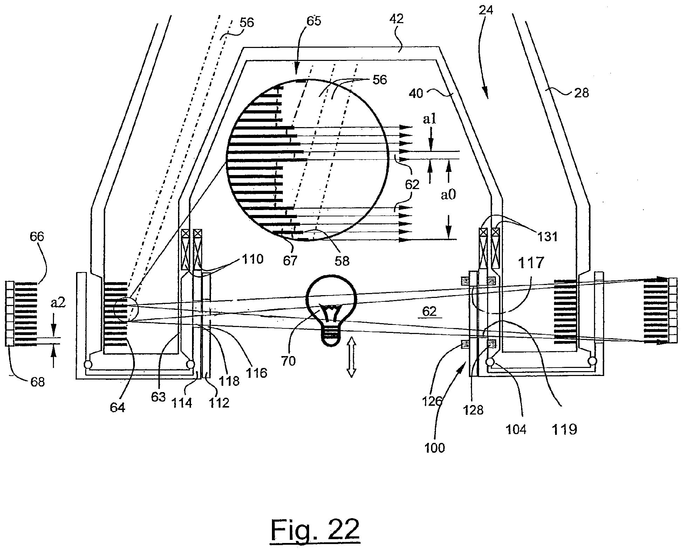

In Figur 22 ist ein abgewandeltes Tomographiegerät dargestellt, bei welchem die Röntgenbeugungsstruktur 64 und der Targetring 58 zu einem Bauteil zusammengefasst sind. Die Lamellen der Röntgenbeugungsstruktur 64 sind unter einem Gitterabstand a1 angeordnet und bestehen vorzugsweise, genauso wie das Target 58, aus einem Material hoher Dichte und hoher Ordnungszahl, weshalb die Oberfläche der Beugungsstruktur prinzipiell gut zur Erzeugung von Röntgenstrahlen geeignet ist.

Vorzugsweise sind bei einer solchen Kombieinheit 65 von Target und Röntgenbeugungsstruktur Zwischenräume zwischen den Gitterlamellen mit einem gut wärmeleitenden und wenig Strahlung absorbierenden Material 67 ausgefüllt, welches zugleich eine Röntgen-Filterwirkung haben kann.

Die für die Kohärenz des Quellpunktes notwendige Strukturierung wird durch die in der Kombieinheit 65 dargestellte, hervorstehenden Gruppen von Lamellen erzeugt, welche unter einem Abstand a0 angeordnet sind. Durch das gruppenweise Vorstehen der Lamellen wird der schräg von oben anfallende Elektronenstrahl in den Zwischenbereichen abgeschattet, weshalb dort keine Röntgenstrahlung mehr erzeugt wird. Durch die dargestellte Sheddach-ähnliche innere Begrenzungsfläche des Lamellenstapels wird die Intensität des Elektronenstrahles 56 besser genutzt. Bei dieser Variante kann die Durchstrahlung des Objektes, im Gegensatz zu den Varianten gemäß den Figuren 2 bis 6 in einer exakten Ebene erfolgen.

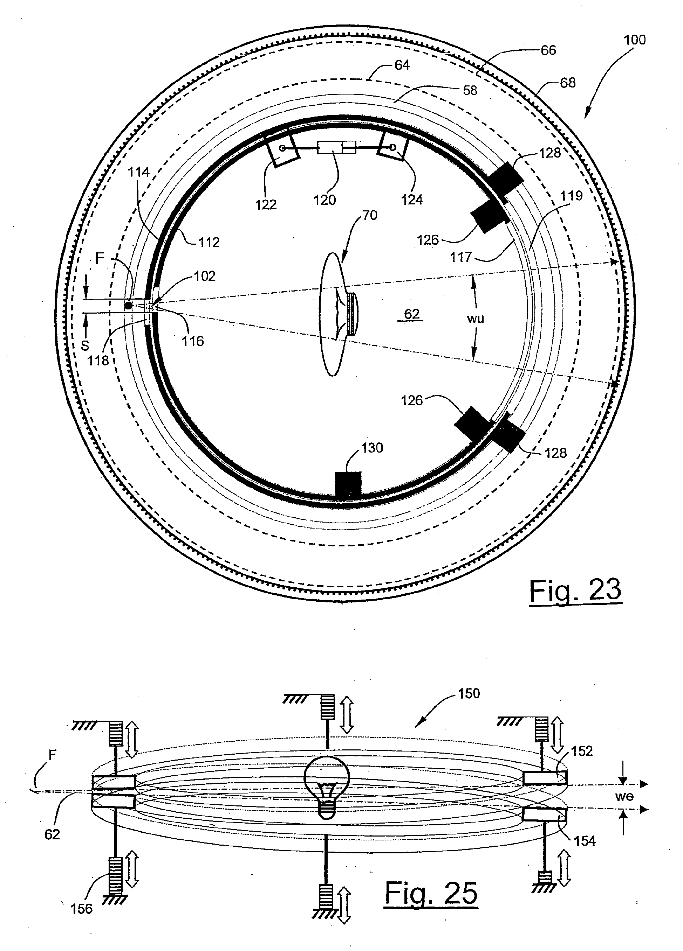

Zusätzlich ist in Figur 22 und 23 eine insgesamt mit 100 bezeichnete rotierende Blendeneinrichtung dargestellt, die zur Funktion des Tomographiegerätes bei technischen Objekten nicht notwendig ist, jedoch zur Senkung der Strahlenbelastung für Patienten im medizinischen Einsatz sinnvoll ist. Sie umfasst Bauteile 100 bis 131.

Der Röntgenstrahlfächer 62 wird dabei durch gegeneinander verstellbare Röntgenfenster 116, 118 auf die notwendige Grenze des Winkels wu und we begrenzt. Eine Lagerung 104 der Umlaufblende ist durch ein großen Durchmesser aufweisendes Kugellager am Boden 30 des Gehäuses 24 realisiert, wobei hier auch eine Luftkissenlagerung oder eine Magnetlagerung verwendbar wäre.

Ein Antrieb 110 und eine Positionsmeldung 131 sorgen für die gewünschte dynamische Positionierung zum Röntgenemissionspunkt F.

Die Blende 102 besteht aus zwei zylindrischen Blendenteilen 112, 114, welche mit Ausnahme von für Röntgenlicht durchlässigen Fenstern 116 bis 119 für Röntgenlicht undurchlässig sind.

Die Fenster 116 und 118 begrenzen so zusammen einen Spalt S, der durch Verdrehen eines der Blendenteile 112, 114 auf dem anderen einstellbar ist, den in Richtung Objekt austretenden Röntgenstrahlfächer in Umfangsrichtung. Die Blendenteile 112, 114 können zum Beispiel durch manuelles Verstellen einer Langlochverbindung in Umfangsrichtung gegen einander verstellt werden, um die Breite des Spaltes S zu verändern.

Alternativ kann man die beiden Blendenteile 112, 114 auch über einen in Umfangsrichtung wirkenden Aktor 120 koppeln, der an Armen 122, 124 angreift, welche am Blendenteil 112 bzw. 114 angebracht sind.

Wuchtkörper 126 und 128, die jeweils zu beiden Seiten der Fenster 116, 118 symmetrisch angeordnet sind, dienen zum Massenausgleich für die leichteren Fenster 116, 118.

Ein weiterer Wuchtkörper 130 ist diametral der Verstelleinrichtung 120, 122, 124 gegenüberliegend auf dem inneren Blendenteil 112 angeordnet und gleicht die Unwucht aus, welche durch die Verstelleinrichung erzeugt wird.

Der Aktor 120 wird drahtlos mit Energie versorgt und drahtlos (ggf. über die gleiche Übertragungsstrecke) ferngesteuert.

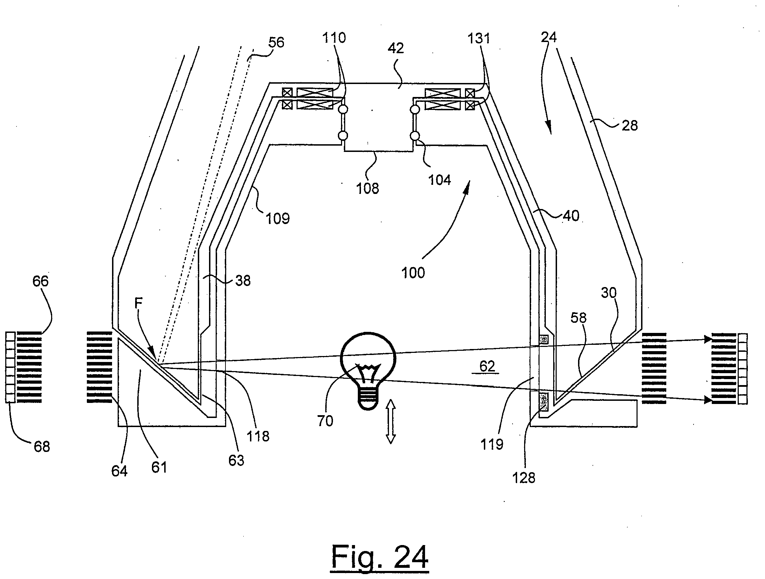

Bei Verwendung der oben beschriebenen Blende 102 kann man über den Spalt S die transversale Abmessung des Röntgenlichtes 62 in der Untersuchungsebene vorgeben. In Figur 24 ist eine weitere Variante dargestellt, mit der ebenfalls eine Durchstrahlung des Objektes in einer exakten Ebene erfolgen kann.

Hier wird der Targetring 58 und der Gehäuseboden 30 so dimensioniert, dass der Röntgenfächer diesen auf der gegenüberliegenden Seite durchstrahlen kann.

In Figur 24 ist zusätzlich eine Abwandlung der rotierenden Blende mit einer Aufhängung 109 in Form einer Glocke oder Speichenkrone dargestellt, deren Lagerung 104 sich an einer zentralen Achse 108, welche an der Endwand 42 des Gehäuses 24 befestigt ist, befindet. Auf dem dem Emissionspunkt F benachbarten Bereich der rotierenden Blende befindet sich zusätzlich ein mitrotierender Abschirmkörper 61, der die Strahlungsemission in die dem Emissionspunkt F benachbarten Detektorbereiche beschränkt.

Die Auswertung der Ausgangssignale der Detektorzeile 68 erfolgt bei Verwendung der Blendenkörper nach Figur 22 bis Figur 24 so, dass man jeweils einen Teilbereich der Detektorzeile 68 scharf schaltet bzw. deren Ausgangssignale auswertet, auf welchem nutzbare Interferenzmuster erhalten werden. Das Auswählen dieses Bereiches erfolgt unter Verwendung des Stellungsgebers 131 oder auch unter Auswertung der Ablenksignale für die Ablenkspule 54.

Figur 25 zeigt eine Blende 150, welche den Öffnungswinkel des Röntgenfächers in Elevationsrichtung (Öffnungswinkel in axialer Schnittebene) begrenzt.

Ein oberer Blendenring 152 und ein unterer Blendenring 154 sind über jeweils wenigstens drei Aktoren (Hubmagnete, Schwingmagnete, Piezosteller, Pneumatikzylinder, Magneto striktive Aktoren) 156 an geeigneter Stelle des Gehäuses 24 befestigt, wobei gegebenenfalls auch einer der beiden Blendenringe 152, 154 feststehend angebracht sein kann. Durch eine phasenverschobene sinusartige Ansteuerung der Aktoren kann nun die entsprechende Blende eine Taumelbewegung ausführen, wodurch der Elevationswinkel we des Röntgenfächers 62 lokal begrenzt wird und gleichzeitig dafür gesorgt wird, dass die Strahlung im Röntgenfächer 62, welche den Patienten durchdringt, auch auf dem Detektor registriert werden kann. Hierdurch lässt sich die Strahlenbelastung für den Patienten reduzieren.

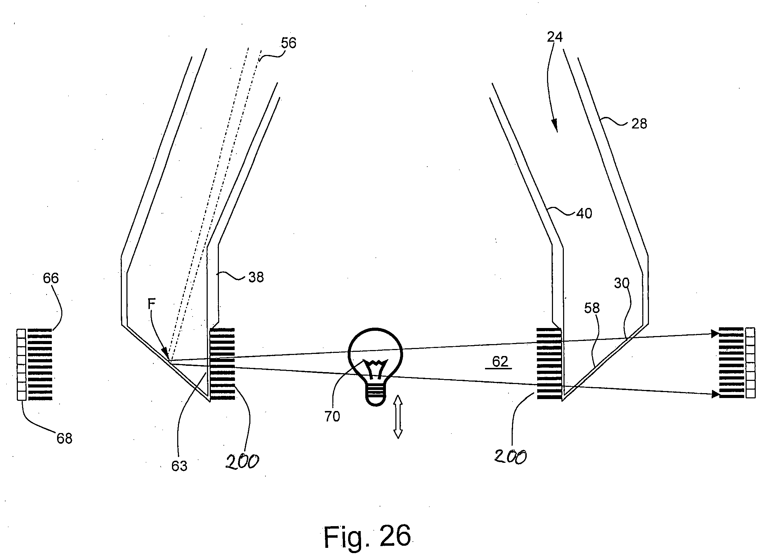

Figur 26 zeigt ein Phasenkontrast-Röntgen-Tomographiegerät nach einem weiteren Ausführungsbeispiel der Erfindung.

Bei diesem Phasenkontrast-Röntgen-Tomographiegerät sind die Bodenwand 30 des evakuierten Gehäuses 24, auf welcher das kegelstumpfförmige Target 58 angeordnet ist, sowie das Target 58 selbst aus einem für das Röntgenlicht durchlässigen Material gebildet. Der Öffnungswinkel des kegelstumpfförmigen Targets 58 ist zudem so gewählt, dass das Röntgenlicht, nachdem es aus dem Austrittsfenster 63 ausgetreten ist und den Untersuchungsbereich durchlaufen hat, auf der diametral gegenüberliegenden Seite wieder auf das Austrittsfenster 63 trifft.

Vom Austrittsfensters 63 aus gesehen radial innenliegend ist ein den Untersuchungsbereich umgebendes Gitter 200 angeordnet, welches zugleich die Funktion des Kohärenzgitters 12 und des Phasengitters 64 übernimmt. Radial außerhalb der Bodenwand 30 sind das Amplitudengitter 66 und der Detektorstreifen 20 angeordnet, deren Funktionsweise gegenüber den bisherigen Ausführungsbeispielen unverändert ist.

Im Übrigen funktioniert das Phasenkontrast-Röntgentomographiegerät wie folgt:
Das am Brennfleck F auf dem Target 58 erzeugte Röntgenlicht trifft nachdem es aus dem Austrittsfenster 63 ausgetreten ist zunächst auf das Gitter 200, wodurch Röntgenlicht mit für die Phasenmessung ausreichender Kohärenz erzeugt wird.

Nach dem Durchlaufen des Untersuchungsbereiches trifft das Röntgenlicht wieder auf das Gitter 200 und tritt durch dieses in entgegengesetzter Richtung hindurch. Das Gitter 200 dient nun als Teil der Phasenmesseinrichtung zusammen mit dem Amplitudengitter 66 und dem Detektorstreifen 20 dazu, die Phasenlage des Röntgenlichts zu erfassen. Das Röntgenlicht tritt dabei über das Austrittsfenster 63 wieder in das Gehäuse 24 ein, um daraufhin durch das Target 58 und die Bodenwand 30 hindurch zu treten. Dort trifft es schließlich auf das Amplitudengitter 66 und den Detektorstreifen 20.

Das soeben beschriebene Ausführungsbeispiel erlaubt es somit, mit nur zwei Gittern ein Phasenkontrast-Röntgen-Tomographiegerät zu realisieren, das zum Erfassen der einzelnen Tomographieprojektionen keine um das zu untersuchende Objekt herum bewegten Teile benötigt. Würde ein Detektorstreifen 20 mit einer ausreichend hohen Auflösung (beispielsweise mit der Auflösung des Amplitudengitters 66) verwendet, so könnte zudem auf das Amplitudengitter 66 verzichtet werden.

In Kombination mit dem Ausführungsbeispiel nach Figur 22 wäre sogar ein Phasenkontrast-Röntgen-Tomographiegerät denkbar, bei welchem nur ein mit einer Gitterstruktur versehenes Target und ein hochauflösender Detektor verwendet werden.

## Patentansprüche

1. Phasenkontrast-Röntgen-Tomographiegerät mit einer Röntgenlichtquelle, die mindestens einen in einem Vakuumgefäß (24) der Röntgenlichtquelle ablenkbaren Elektronenstrahl (56) aufweist, der auf einer vorgegebenen Bahn (K) über ein Target (58) der Röntgenlichtquelle bewegbar ist, welches einen Untersuchungsbereich zumindest teilweise umgibt, um auf dem Target (58) mindestens einen Röntgenlicht emittierenden Brennfleck (F) zu erzeugen,
mit einer Röntgenlicht-Detektionseinheit, welche eine feststehende Röntgenlicht-Phasenmesseinrichtung (64, 66, 68) umfasst, die vom Röntgenlicht bei dessen Bewegung überstrichen wird, und
mit einer Auswerteeinrichtung (86), die mit den Ausgangssignalen der Phasenmesseinrichtung (64, 66, 68) beaufschlagt ist und dazu eingerichtet ist, aus diesen ein durch Phasenkontrast erhaltenes Objektbild zu berechnen.

2. Phasenkontrast-Röntgen-Tomographiegerät nach Anspruch 1, **dadurch gekennzeichnet, dass** der Elektronenstrahl (56) in seiner Intensität modulierbar ist.

3. Phasenkontrast-Röntgen-Tomographiegerät nach einem der Ansprüche 1 bis 2, **dadurch gekennzeichnet, dass** die radiale Position des Elektronenstrahles (56) variabel ist, vorzugsweise in Abhängigkeit von der Größe eines zu durchstrahlenden Bereiches.

4. Phasenkontrast-Röntgen-Tomographiegerät nach einem der Ansprüche 1 bis 3, **dadurch gekennzeichnet, dass** das Target (58) zumindest teilweise rotationssymmetrisch ist, wobei es vorzugsweise die Form eines sich in Richtung zu einer Elektronenstrahlquelle (44) der Röntgenlichtquelle erweiterten Kegelstumpfes oder eines Teiles eines solchen hat, der vorzugsweise einen derartigen Öffnungswinkel aufweist, dass das Bild des Brennfleckes (F) einem Talbot-Interferometer (64, 66) der Phasenmesseinrichtung (64, 66, 68) angepasst ist.

5. Phasenkontrast-Röntgen-Tomographiegerät nach einem der Ansprüche 1 bis 4, **dadurch gekennzeichnet, dass** die Phasenmesseinrichtung (64, 66, 68) einen Detektor (68) umfasst, der als geschlossener Ring ausgebildet ist, der vorzugsweise kreisförmig oder polygonförmig ist.

6. Phasenkontrast-Röntgen-Tomographiegerät nach einem der Ansprüche 1 bis 5, **dadurch gekennzeichnet, dass** die Phasenmesseinrichtung (64, 66, 68) einen Detektor (68) umfasst, der eine mehrzeilige Anordnung von Detektorelementen (68i-j) umfasst, deren Zeilenhöhe vorzugsweise einen Bruchteil der Pixelauflösung beträgt.

7. Phasenkontrast-Röntgen-Tomographiegerät nach einem der Ansprüche 1 bis 6, **dadurch gekennzeichnet, dass** in Strahlrichtung gesehen hinter der Röntgenlichtquelle ein Röntgengitter (12; 200) als Kohärenzgitter angeordnet ist.

8. Phasenkontrast-Röntgen-Tomographiegerät nach Anspruch 7, **dadurch gekennzeichnet, dass** das Röntgengitter (200) den Untersuchungsbereich zumindest teilweise umgibt, wobei das Röntgengitter (200) in Strahlrichtung des Röntgenlichts gesehen vor dem Untersuchungsbereich als Kohärenzgitter und in Strahlrichtung des Röntgenlichts gesehen hinter dem Untersuchungsbereich als Röntgen-Beugungsstruktur der Phasenmesseinrichtung (200, 66, 68) wirkt.

9. Phasenkontrast-Röntgen-Tomographiegerät nach einem der Ansprüche 1 bis 8, **dadurch gekennzeichnet, dass** die Phasenmesseinrichtung eine Interferenzeinrichtung (64, 66) und einen Detektor (68) umfasst, wobei die Interferenzeinrichtung mindestens eine Röntgenlicht-Beugungsstruktur (64, 66) aufweist, welche in Strahlrichtung gesehen vor dem Detektor (68) der Phasenmesseinrichtung (64, 66, 68) angeordnet ist.

10. Phasenkontrast-Röntgen-Tomographiegerät nach Anspruch 9, **dadurch gekennzeichnet, dass** die Interferenzeinrichtung (64, 66) zwei in Strahlrichtung beabstandete Röntgenlicht-Beugungsstrukturen (64, 66), vorzugsweise Röntgengitter, umfasst, welche vorzugsweise unter Talbot-Abstand in Strahlrichtung beabstandet angeordnet sind.

11. Phasenkontrast-Röntgen-Tomographiegerät nach einem der Ansprüche 9 bis 10, **dadurch gekennzeichnet, dass** mindestens eine der Röntgenlicht-Beugungsstrukturen (64, 66) als Ring (76) oder Ringsegment (82) ausgebildet ist und vorzugsweise kreisförmige oder polygonale Grundgeometrie aufweist.

12. Phasenkontrast-Röntgen-Tomographiegerät nach einem der Ansprüche 11, **dadurch gekennzeichnet, dass** die einem Objekt am nächsten liegende Beugungsstruktur (64) auf ihrer dem Objekt zugewandten Seite eine überlagerte doppel-periodische Beugungsstruktur (65) mit einer ersten Gitterkonstanten (a1), die der Talbotbedingung für diese Beugungsstruktur (64) genügt, und einer zweiten periodischen Abstandskonstanten (a0), die der Talbotbedingung für die Kohärenz des Quellpunktes genügt, und durch Abschattungsbereiche gegenüber dem einfallenden Elektronenstrahl (56) ausgebildet ist.

13. Phasenkontrast-Röntgen-Tomographiegerät nach einem der Ansprüche 1 bis 12, **gekennzeichnet durch** Mittel (146) zum Verändern einer oder mehrerer Relativlagen zwischen einem oder mehreren der nachstehenden Komponenten bzw. Teilen hiervon einerseits und einer anderen der nachstehenden Komponenten andererseits: Röntgenlichtquelle, insbesondere der Brennfleck (F); Kohärenzgitter; Phasenmesseinrichtung, insbesondere die Röntgenlicht-Beugungsstrukturen (64, 66) und/oder der Detektor (68).

14. Phasenkontrast-Röntgen-Tomographiegerät nach einem der Ansprüche 1 bis 13, **dadurch gekennzeichnet, dass** die Röntgenlichtquelle einen Austrittsspalt (60; S; 116, 118, 134, 141) für das Röntgenlicht aufweist, der in einer senkrecht zu einer Geräteachse liegenden Ebene verläuft und vorzugsweise durch eine zumindest teilweise kreisförmige oder polygonförmige Blende (38, 102, 112, 114, 140, 142) begrenzt ist und vorzugsweise einen an die entsprechende Detektorabmessung angepassten Elevations-Öffnungswinkel (we) für das Röntgenlicht (62) und/oder einen derart angepassten Umfangs-Öffnungswinkel (wu) vorgibt.

15. Phasenkontrast-Röntgen-Tomographiegerät nach einem der Ansprüche 1 bis 14, **gekennzeichnet durch** eine Mehrzahl in Umfangsrichtung aufeinanderfolgende Blendenfenster (134).

## Claims

1. Phase contrast x-ray tomography device
having an x-ray light source, which has at least one electron beam (56) deflectable in a vacuum vessel (24) of the x-ray light source, wherein the electron beam (56) can be moved on a predetermined path (K) over a target (58) of the x-ray light source, which at least partially surrounds an examination region, in order to generate at least one focal spot (F) emitting x-ray light on the target (58),
having an x-ray-light detection unit, which comprises a stationary x-ray light phase measuring device (64, 66, 68), which is scanned by the x-ray light upon its movement, and
having an evaluating device (86), which is supplied with the output signals of the phase measuring device (64, 66, 68) and is configured to calculate from these signals an object image obtained by phase contrast.

2. Phase contrast x-ray tomography device according to Claim 1, **characterized in that** the electron beam (56) can be modulated in its intensity.

3. Phase contrast x-ray tomography device according to one of Claims 1 to 2, **characterized in that** the radial position of the electron beam (56) is variable, preferably in dependence on the size of a region to be radiographed.

4. Phase contrast x-ray tomography device according to one of Claims 1 to 3, **characterized in that** the target (58) is at least partially rotationally symmetrical, wherein it preferably has the shape of a truncated cone or of a part of such, widening in the direction towards an electron beam source (44) of the x-ray light source, and preferably having such an opening angle that the image of the focal spot (F) is adapted to a Talbot interferometer (64) of the phase measuring device (64, 66, 68).

5. Phase contrast x-ray tomography device according to one of Claims 1 to 4, **characterized in that** the phase measuring device (64, 66, 68) comprises a detector (68) which is formed as a closed ring, which is preferably circular or polygonal.

6. Phase contrast x-ray tomography device according to one of Claims 1 to 5, **characterized in that** the phase measuring device (64, 66, 68) comprises a detector (68), which comprises a multi-line arrangement of detector elements (68i-j), the line height of which is preferably a fraction of the pixel resolution.

7. Phase contrast x-ray tomography device according to one of Claims 1 to 6, **characterized in that**, seen in the beam direction behind the x-ray light source, there is arranged an x-ray grating (12; 200) as a coherence grating.

8. Phase contrast x-ray tomography device according to Claim 7, **characterized in that** the x-ray grating (200) at least partially surrounds the examination region, wherein the x-ray grating (200), seen in the beam direction of the x-ray light in front of the examination region, acts as a coherence grating and, seen in the beam direction of the x-ray light behind the examination region, acts as an x-ray diffraction structure of the phase measuring device (200, 66, 68).

9. Phase contrast x-ray tomography device according to one of Claims 1 to 8, **characterized in that** the phase measuring device comprises an interference device (64, 66) and a detector (68), wherein the interference device has at least one x-ray-light diffraction structure (64, 66) which, seen in the beam direction, is arranged in front of the detector (68) of the phase measuring device (64, 66, 68).

10. Phase contrast x-ray tomography device according to Claim 9, **characterized in that** the interference device (64, 66) comprises two x-ray-light diffraction structures (64, 66), preferably x-ray gratings, spaced in the beam direction, which are preferably arranged at Talbot spacings spaced in the beam direction.

11. Phase contrast x-ray tomography device according to one of Claims 9 to 10, **characterized in that** at least one of the x-ray-light diffraction structures (64, 66) is formed as a ring (76) or ring segment (82) and preferably has a circular or polygonal basic geometry.

12. Phase contrast x-ray tomography device according to one of Claims 11, **characterized in that** the diffraction structure (64) lying nearest an object, has, on its side facing the object, a superposed double-periodic diffraction structure (65) with a first grating constant (a1) which satisfies the Talbot condition for this diffraction structure (64), and a second periodic spacing constant (a0) which satisfies the Talbot condition for the coherence of the source point, and is formed by shading regions with respect to the incident electron beam (56).

13. Phase contrast x-ray tomography device according to one of Claims 1 to 12, **characterized by** means (146) for changing one or more relative positions between one or more of the following components or parts thereof on one hand, and, on the other hand, another of the following components: x-ray light source, in particular focal spot (F); coherence grating; phase measuring device, in particular the x-ray-light diffraction structures (64, 66) and/or the detector (68).

14. Phase contrast x-ray tomography device according to one of Claims 1 to 13, **characterized in that** the x-ray light source has an outlet gap (60; S; 116, 118, 134, 141) for the x-ray light, which runs in a plane lying perpendicular to a device axis and is preferably limited by an at least partially circular or polygonal aperture (38, 102, 112, 114, 140, 142) and preferably presents an elevation opening angle (we) for the x-ray light (62) which is adapted to the corresponding detector dimension, and/or a circumferential opening angle (wu) adapted in such a manner.

15. Phase contrast x-ray tomography device according to one of Claims 1 to 14, **characterized by** a plurality of aperture windows (134) follow one another in the circumferential direction.

## Revendications

1. Appareil de tomographie par rayons X à contraste de phase,
comportant une source de rayonnement X qui présente au moins un faisceau d'électrons (56) qui peut être dévié dans un récipient sous vide (24) de la source de rayonnement X et peut être déplacé le long d'une trajectoire prédéterminée (K) sur une cible (58) de la source de rayonnement X qui entoure au moins partiellement une zone d'examen afin de générer au moins un point focal (F) émettant un rayonnement X sur la cible (58), comportant une unité de détection de rayonnement X qui comprend un dispositif de mesure de phase de rayonnement X fixe (64, 66, 68) qui est balayé par le rayonnement X lors de son déplacement et
comportant un dispositif d'évaluation (86) qui est soumis aux signaux de sortie du dispositif de mesure de phase (64, 66, 68) et conçu pour calculer à partir de ceux-ci une image objet obtenue par contraste de phase.

2. Appareil de tomographie par rayons X à contraste de phase selon la revendication 1, **caractérisé en ce que** l'intensité du faisceau d'électrons (56) est modulable.

3. Appareil de tomographie par rayons X à contraste de phase selon l'une des revendications 1 à 2, **caractérisé en ce que** la position radiale du faisceau d'électrons (56) est variable, de préférence en fonction de la taille d'une zone à radiographier.

4. Appareil de tomographie par rayons X à contraste de phase selon l'une des revendications 1 à 3, **caractérisé en ce que** la cible (58) est au moins partiellement symétrique en rotation, de préférence présente la forme d'un cône tronqué ou d'une partie de cône tronqué qui s'élargit en direction d'une source de faisceau d'électrons (44) de la source de rayonnement X et présente de préférence un angle d'ouverture tel que l'image du point focal (F) est adaptée à un interféromètre de Talbot (64, 66) du dispositif de mesure de phase (64, 66, 68).

5. Appareil de tomographie par rayons X à contraste de phase selon l'une des revendications 1 à 4, **caractérisé en ce que** le dispositif de mesure de phase (64, 66, 68) comprend un détecteur (68) qui est réalisé sous la forme d'un anneau fermé, de préférence circulaire ou polygonal.

6. Appareil de tomographie par rayons X à contraste de phase selon l'une des revendications 1 à 5, **caractérisé en ce que** le dispositif de mesure de phase (64, 66, 68) comprend un détecteur (68) qui comprend un agencement multiligne d'éléments détecteurs (68i-j) dont la hauteur de ligne est de préférence une fraction de la résolution en pixels.

7. Appareil de tomographie par rayons X à contraste de phase selon l'une des revendications 1 à 6, **caractérisé en ce qu'**une grille à rayons X (12 ; 200) est disposée en tant que grille de cohérence après la source de rayonnement X, vu dans la direction du faisceau.

8. Appareil de tomographie par rayons X à contraste de phase selon la revendication 7, **caractérisé en ce que** la grille à rayons X (200) entoure au moins partiellement la zone d'examen, la grille à rayons X (200) agissant comme grille de cohérence avant la zone d'examen, vu dans la direction de faisceau du rayonnement X, et comme structure de diffraction de rayons X du dispositif de mesure de phase (200, 66, 68) après la zone d'examen, vu dans la direction de faisceau du rayonnement X.

9. Appareil de tomographie par rayons X à contraste de phase selon l'une des revendications 1 à 8, **caractérisé en ce que** le dispositif de mesure de phase comprend un dispositif d'interférence (64, 66) et un détecteur (68), le dispositif d'interférence présentant au moins une structure de diffraction de rayons X (64, 66) qui est disposée avant le détecteur (68) du dispositif de mesure de phase (64, 66, 68), vu dans la direction de faisceau.

10. Appareil de tomographie par rayons X à contraste de phase selon la revendication 9, **caractérisé en ce que** le dispositif d'interférence (64, 66) comprend deux structures de diffraction de rayons X (64, 66), de préférence des grilles à rayons X, qui sont espacées dans la direction de faisceau et sont de préférence espacées l'une de l'autre de la distance de Talbot dans la direction de faisceau.

11. Appareil de tomographie par rayons X à contraste de phase selon l'une des revendications 9 à 10, **caractérisé en ce qu'**au moins une des structures de diffraction de rayons X (64, 66) est réalisée sous la forme d'un anneau (76) ou d'un segment d'anneau (82) et présente de préférence une géométrie de base circulaire ou polygonale.

12. Appareil de tomographie par rayons X à contraste de phase selon l'une des revendications 11, **caractérisé en ce que** la structure de diffraction (64) la plus proche d'un objet présente sur sa face tournée vers l'objet une structure de diffraction superposée à double périodicité (65) avec une première constante de grille (a1), qui satisfait à la condition de Talbot pour cette structure de diffraction (64), et une deuxième constante d'espacement périodique (a0) qui satisfait à la condition de Talbot pour la cohérence du point source et qui est formée par des zones d'occultation vis-à-vis du faisceau d'électrons incident (56).

13. Appareil de tomographie par rayons X à contraste de phase selon l'une des revendications 1 à 12, **caractérisé par** des moyens (146) pour modifier une ou plusieurs positions relatives entre un ou plusieurs des composants suivants ou parties de ceux-ci d'une part et un autre des composants suivants d'autre part : source de rayonnement X, en particulier le point focal (F) ; grille de cohérence ; dispositif de mesure de phase, en particulier les structures de diffraction de rayonnement X (64, 66) et/ou le détecteur (68).

14. Appareil de tomographie par rayons X à contraste de phase selon l'une des revendications 1 à 13, **caractérisé en ce que** la source de rayonnement X présente une fente de sortie (60 ; S ; 116, 118, 134, 141) pour le rayonnement X, qui s'étend dans un plan perpendiculaire à un axe de l'appareil, est de préférence limitée par un diaphragme au moins partiellement circulaire ou polygonal (38, 102, 112, 114, 140, 142) et prédéfinit de préférence pour le rayonnement X (62) un angle d'ouverture en élévation (we) adapté à la dimension correspondante du détecteur et/ou un angle d'ouverture circonférentiel (wu) adapté d'une telle façon.

15. Appareil de tomographie par rayons X à contraste de phase selon l'une des revendications 1 à 14, **caractérisé par** une pluralité de fenêtres de diaphragme (134) se succédant dans la direction circonférentielle.
